# EUROPEAN PATENT SPECIFICATION

(11) **EP 2 693 938 B1**
(45) Date of publication and mention of the grant of the patent: **12.02.2020**
(21) Application number: 12722939.1
(22) Date of filing: 03.03.2012
(51) Int. Cl.: A61B 5/0476

(54) **DETECTING, ASSESSING AND MANAGING EXTREME EPILEPTIC EVENTS**
ERKENNUNG BEWERTUNG UND VERWALTUNG VON EXTREMEN EPILEPTISCHEN EREIGNISSEN
DÉTECTION, ÉVALUATION ET GESTION D'ÉVÈNEMENTS ÉPILEPTIQUES EXTRÊMES

(30) Priority: 04.03.2011 US 201113040996; 20.04.2011 US 201113091033; 21.12.2011 US 201113333235
(43) Date of publication of application: 12.02.2014
(73) Proprietor: Flint Hills Scientific, LLC, Lawrence, KS 66044-0189 (US)
(72) Inventor: OSORIO, Ivan, Leawood, Kansas 66209 (US)
(74) Representative: Grünecker Patent- und Rechtsanwälte PartG mbB
(86) International application number: PCT/US2012/027639
(87) International publication number: WO 2012/122066

(56) References cited:
- US-A1- 2008 033 508
- US-A1- 2010 274 303
- OSORIO I ET AL: "Toward a quantitative multivariate analysis of the efficacy of antiseizure therapies", EPILEPSY AND BEHAVIOR, ACADEMIC PRESS, SAN DIEGO, CA, US, vol. 18, no. 4, 1 August 2010 (2010-08-01) , pages 335-343, XP027210402, ISSN: 1525-5050 [retrieved on 2010-06-11]

## Description

### FIELD OF THE INVENTION

This invention relates generally to medical device systems and, more particularly, to medical device systems and methods capable of assessing and managing extreme events related to seizures, e.g., seizures resulting from epilepsy.

### DESCRIPTION OF THE RELATED ART

Generalized tonic-clonic status epilepticus, referred to herein as Convulsive Status Epilepticus (CSE) is a neurological emergency with an estimated incidence of about 20 out of 100,000 patients and is generally considered an extreme event. CSE is also associated with a mortality rate between 3% and 40% depending on etiology, age, status type, and status duration. CSE, in particular, requires immediate, aggressive, and effective treatment to stop seizure activity, to prevent neuronal damage, systemic complications and the possibility of death. Most investigations on prognosis of status epilepticus (SE) have focused on mortality, and some research suggests that outcome basically depends on the etiological and biological background but also that the earlier the therapeutic intervention the higher the probability of controlling it. Additionally, non-convulsive status epilepticus (nCSE), while not a medical emergency of the magnitude of CSE, it is also an extreme event as nevertheless it increases the risk of bodily injury and neurologic deficits such as permanent potentially severe impairment in memory.

CSE and nCSE are described based on the duration of a single seizure and its variations or on the lack of recovery of certain neurologic functions to their inter-ictal (baseline) levels in the context of closely spaced seizures. The focus on seizure duration or frequency or on level of consciousness or of awareness to determine if a patient is in status epilepticus has important limitations, since signals or indices from others systems such as cardio-vascular, respiratory, endocrine and metabolic which are also adversely impacted by the seizures and which directly contribute to the increased (compared to the non-epileptic population) morbidity and mortality of patients with epilepsy are disregarded. The state of the art views and treats Status Epilepticus narrowly and ineffectively. Embodiments of this invention takes a system's approach by quantifying the impact of seizures on bodily functions (e.g., neurologic, cardiovascular) to determine the probability they are harbinger of extreme events (e.g., status epilepticus) and to prevent them from occurring, or if they are extreme, to provide early treatment and/or warning to avert serious neurological and medical sequelae or even fatal outcomes.

Sudden Unexpected Death in Epilepsy, or "SUDEP," another extreme event, is a phenomenon in which a patent with epilepsy dies unexpectedly and without an apparent, outstanding cause, that is, the death is unexplained since autopsy results are unrevealing.

CSE and nCSE alter autonomic nervous system function. Since brain/neurological activity such as electrical activity, whether normal or abnormal, and autonomic functions (e.g., cardiovascular activity, respiration, etc.), referred to herein as body signals (from which body data may be derived), are functionally tightly coupled; monitoring these body signal provides valuable information. This is the first invention to utilize not only neurologic, autonomic, metabolic, endocrine and tissue stress marker signals but do so in a multi-variant adaptive manner to optimize sensitivity and specificity of detection of extreme epileptic events (e.g., CSE, Sudden Unexpected Death in Epilepsy (SUDEP)), and, more importantly to anticipate them.

US 2010/274303 A1 discloses a method for the detection of epilepsy events including a threshold comparison based on the intensity of the seizure and taking medical action in response to the intensity of the measure.

US 2008/033508 discloses a method for detecting epileptic events using non-linear analysis parameters wherein, in particular, non linear analysis parameters are compared to thresholds.

I. Osorio et al., in a paper entitled "Toward a quantitative multivariate analysis of the efficacy of antiseizure therapy", Epilepsy and Behaviour, Academic Press, San Diego, CA, US, vol. 18, no. 4, 1 August 2010, pages 335-343, discloses a study on the efficacy of antiseizure therapy based the measurement of intensity, duration and spread of seizures and time between seizures.

### SUMMARY OF EMBODIMENTS

It is provided a computer implemented method for identifying an extreme seizure event in a patient, comprising:
determining at least one of an autonomic index, a neurologic index, a metabolic index, an endocrine index, or a tissue stress index, said at least one determined index being based upon body data;
identifying a seizure event based upon said at least one determined index;
determining at least one seizure severity index, SSI, value indicative of the severity of said seizure event based on the product of the duration of the seizure event determined as the time that any of the values of the autonomic index, neurologic index, metabolic index, endocrine index, or tissue stress index differ from inter-ictal and/or post-ictal values and the peak intensity of the seizure event defined by a maximum value of the body data;
comparing said determined at least one SSI value to at least one extreme reference value, wherein the extreme reference value is a value above the ninetieth percentile of a plurality of past SSI values over a first time period; and
identifying an occurrence of an extreme seizure event, based upon the comparison of said determined SSI value to said at least one extreme reference value;
and wherein said extreme seizure event comprises at least one of a status epilepticus event or a pathophysiological effect resulting from an extreme epileptic state, the pathophysiological effect being selected from the group consisting of:
   damage to brain tissue resulting in permanent/serious motor/visual/sensory/cognitive skills; respiratory failure, cardiac failure, pulmonary edema, cardiac arrhythmia, arterial blood acidosis, liver/renal failure, bed sores, bone fractures, abrasions, bruises, organ failure, multi-organ failure, arterial hypertension, tissue hypoxia and tissue acidosis.

Furthermore, it is provided an apparatus, comprising:
a determination component adapted to:
   determine at least one of an autonomic index, a neurologic index, a metabolic index, an endocrine index, or a tissue stress index, said at least one determined index being based upon body data;
   identify a seizure event based upon said at least one determined index;
   determine an SSI value indicative of the severity of said seizure event based on the product of the duration of the seizure event determined as the time that any of the values of the autonomic index, neurologic index, metabolic index, endocrine index, or tissue stress index differ from inter-ictal and/or post-ictal values and the peak intensity of the seizure event defined by a maximum value of the body data;
   compare the determined SSI value to at least one extreme reference value, wherein the extreme reference value is a value above the ninetieth percentile of a plurality of past SSI values over a first time period, and
   identify an occurrence of an extreme epileptic event, based upon the comparison of said determined SSI value to said at least one extreme reference value;
and wherein said extreme seizure event comprises at least one of a status epilepticus event or a pathophysiological effect resulting from an extreme epileptic state, the pathophysiological effect being selected from the group consisting of:
   damage to brain tissue resulting in permanent/serious motor/visual/sensory/cognitive skills; respiratory failure, cardiac failure, pulmonary edema, cardiac arrhythmia, arterial blood acidosis, liver/renal failure, bed sores, bone fractures, abrasions, bruises, organ failure, multi-organ failure, arterial hypertension, tissue hypoxia and tissue acidosis.

### BRIEF DESCRIPTION OF THE DRAWINGS

The present invention may be understood by reference to the following description taken in conjunction with the accompanying drawings, in which like reference numerals identify like elements, and in which:
Figure 1A provides an exemplary stylized diagram of a medical device which may be implanted into a patient's body for providing a therapeutic electrical signal to a neural structure of the patient's body, in accordance with one illustrative embodiment of the present invention;
Figure 1B provides an exemplary stylized diagram of a medical device which may be implanted into a patient's body for providing a therapeutic electrical signal to a neural structure of the patient's body, in accordance with one illustrative embodiment of the present invention;
Figure 1C provides a stylized diagram of an exemplary medical device which may be implanted into a patient's body for providing a therapeutic electrical signal to a neural structure of the patient's body, in accordance with one illustrative embodiment of the present invention;
Figure 2 illustrates an exemplary medical device for anticipating, detecting, assessing and managing (e.g., treating, warning, logging) extreme events related to epilepsy, in accordance with an illustrative embodiment of the present invention;
Figure 3 provides a stylized diagram of an exemplary medical device and its different data acquisition units that may be implanted into a patient's body, in accordance with one illustrative embodiment of the present invention;
Figure 4 provides a stylized diagram of an exemplary medical device which may be implanted into a patient's body, in accordance with one illustrative embodiment of the present invention;
Figure 5A provides a stylized diagram of a seizure severity index unit for determining a seizure severity index using body data and seizure data, in accordance with one illustrative embodiment of the present invention;
Figure 5B provides a stylized diagram of a patient impact unit for determining a patient impact using body data and seizure data, in accordance with one illustrative embodiment of the present invention;
Figure 5C provides a stylized diagram of an inter-seizure interval index unit for determining a time elapsed between the onset of consecutive or non-consecutive seizures using body data, in accordance with one illustrative embodiment of the present invention;
Figure 6 provides a stylized diagram of a warning unit for warning of a patient's proclivity towards an extreme epileptic event/state or of its occurrence, in accordance with one illustrative embodiment of the present invention;
Figure 7 provides a flowchart depiction of a method for identifying and/or managing an extreme epileptic event/state, in accordance with one illustrative embodiment of the present invention;
Figure 8 provides a flowchart depiction of a method implementing responsive actions (warning, treatment, data logging among others) in response to determining that an extreme epileptic event/state is probable, is occurring or has occurred, in accordance with one illustrative embodiment of the present invention;
Figure 9 provides a flowchart depiction of a method for warning and/or providing a treatment to a patient likely to be in, or to recently have been in an extreme epileptic event/state, in accordance with one illustrative embodiment of the present invention;
Figure 10 illustrates a stylized diagram for determining and executing a treatment plan by a healthcare provider, caregiver and/or patient subsequent to overriding automated treatment an extreme epileptic event/state, in accordance with one illustrative embodiment of the present invention;
Figure 11 provides a stylized diagram of a seizure impact ranking unit adapted to quantify seizure impact based on its effects (type, magnitude and duration) on neurologic, autonomic, metabolic, endocrine, and/or physical integrity (*e.g*., damage to musculoskeletal system), tissue stress markers and/or time spent in seizure per unit time using body data, in accordance with one illustrative embodiment of the present invention;
Figure 12 provides a flowchart depiction of a method for identifying and/or managing a an extreme epileptic event/state, in accordance with one illustrative embodiment of the present invention;
Figure 13 provides a graphical representation of the probability density function of seizure energy estimated from subjects with pharmaco-resistant seizures, in accordance with one illustrative embodiment;
Figure 14A provides a graphical representation one exemplary probability density function of inter-seizure intervals in patients with pharmaco-resistant epilepsies, in accordance with one illustrative embodiment;
Figure 14B provides a graphical depiction of ictal, inter-ictal/inter-seizure, post-ictal and inter-ictal periods, in accordance with one illustrative embodiment;
Figure 15 provides a graphical representation of an increase in seizure likelihood (y-axis) over baseline as a function of time before and after a seizure as determined in a pooled group of patients, according to one illustrative embodiment;
Figure 16A graphically depicts changes in responsiveness/awareness and recovery to baseline as a function of seizure severity in a case on non-extreme events, in accordance with one illustrative embodiment;
Figure 16B depicts an example of an extreme epileptic state where responsiveness/awareness does not recover to baseline in-between seizures due to their severity, in accordance with one illustrative embodiment;
Figure 16C depicts an example of changes in responsiveness/awareness as a function of inter-seizure interval in a non-extreme event case, in accordance with one illustrative embodiment;
Figure 16D depicts an illustrative example of changes in responsiveness/awareness associated with short inter-seizure intervals and compatible with an extreme epileptic event/state as responsiveness remains below baseline;
Figure 17A provides a graphical representation of a seizure measure(s) and/or dimension(s) with more than one threshold (bottom panel), and where said thresholds may be above or below a reference threshold, in accordance with one illustrative embodiment;
Figure 17B depicts that in patients with pharmaco-resistant seizures, the longer the time elapsed from the last seizure, the longer the time until the next seizure, in accordance with one or more embodiments;
Figure 18 provides a stylized diagram of impact of status epilepticus on body organ functions, seizures and quality of life;
Figure 19 provides a stylized depiction of distributions (in percentiles) of values of an exemplary body index chart, during the inter-ictal, ictal and postictal periods, in accordance with one embodiment;
Figure 20 provides a stylized depiction of an exemplary body index chart, including an outlier (*e.g.,* it exceeds the 95^{th} percentile of ictal values) body index value, in accordance with one embodiment;
Figure 21 provides a stylized depiction of the possible distribution (in percentiles) of an exemplary body signal energy chart including an outlier (*e.g.,* the drop in energy exceeds the 5^{th} percentile of postictal values), in accordance with one embodiment; and
Figure 22 provides a stylized depiction of various time periods within distributions (in percentiles) of values of an exemplary body index chart, during the inter-ictal, ictal and post-ictal periods, in accordance with one embodiment.

While the invention is susceptible to various modifications and alternative forms, specific embodiments thereof have been shown by way of example in the drawings and are herein described in detail. It should be understood, however, that the description herein of specific embodiments is not intended to limit the invention to the particular forms disclosed, but on the contrary, the intention is to cover all modifications, equivalents, and alternatives falling within the spirit and scope of the invention as described by the appended claims.

### DETAILED DESCRIPTION OF EMBODIMENTS

Illustrative embodiments of the invention are described herein. In the interest of clarity, not all features of an actual implementation are described in this specification. In the development of any such actual embodiment, numerous implementation-specific decisions must be made to achieve the design-specific goals, which will vary from one implementation to another. It will be appreciated that such a development effort, while possibly complex and time-consuming, would nevertheless be a routine undertaking for persons of ordinary skill in the art having the benefit of this disclosure.

The terms "microscopic," "mesoscopic," and "macroscopic" described herein may show time periods which may be used in observation of seizure events and/or extreme seizure events, body changes such as heart wave and heart wave complex morphology, heart rate variability, and/or other body data described herein. "Microscopic" may correspond to the scale of observation of at least part of a heart beat such as that represented by an EKG's P-QRS-T complex or may correspond to a period of time that is less than a "mesoscopic" time period (*e.g.,* less than 10 seconds). "Mesoscopic" may correspond to a scale of observation of several seconds to tens of seconds (*e.g.,* 10-300 seconds) to capture at least in part, a change in the shape of heart rate plot representative of a state change. "Macroscopic" may correspond to a scale of observation longer than 300 seconds that may be used to encompass more than the information contained in the "mesoscopic" scale or window as described above. In the context of the description provided herein, the term "window" may be used to refer to one or more of the "mesoscopic," "microscopic" and "macroscopic" time periods described above.

A patient may experience certain types of seizures which may be classified as "extreme". As described herein, extreme seizures may be classified based on certain metrics such as a seizure severity index (SSI) and/or inter-seizure interval (ISI), and in other embodiments may be influenced by additional measures, such as the effects of a fall or other injuries associated with a particular seizure event.

Extreme epileptic/seizure events may be classified using the various metrics described herein. In one embodiment, an extreme epileptic/seizure event may be classified at least as one of a generalized seizure or a partial seizure. The term "partial seizure" may encompass complex partial seizures and/or simple partial seizure. Both generalized seizures and partial seizures may result in and/or become extreme epileptic events based on one or more of the criteria described herein. For example, a generalized seizure may result in a seizure severity index (such as heart rate, discussed in further detail below) change being outside a reference range for SSI. The increase in magnitude of the variables that are indicative of an increase in the severity of seizure may lead to a generalized seizure extreme epileptic event. This event may be quantifiable by comparing the SSI to a predetermined reference threshold.

A patient's seizures may be classified as "non-extreme" or as "extreme". As described herein, "extreme" seizures events may be classified based on certain metrics such as a seizure intensity, duration or extent of spread, seizure severity, and/or the impact or burden the extreme seizures have on a subject (also called the patient seizure impact (PSimp)) and/or inter-seizure interval duration. Extreme events may be described quantitatively or qualitatively. Quantitatively, as described herein, they may correspond for example to seizures to the far right (on the *x*-axis) of a seizure severity probability density function, or in the case of a normal/normalized distribution of seizure severity to seizures more than one standard deviation beyond the mean, or as above a percentile of seizure intensity (*e.g*., 80^{th}) or also as those whose conditional probability of occurrence is very low. In the case of inter-seizure intervals (ISIs), extreme events are described quantitatively as those to the far left on an ISI probability density function, or in the case of a normal/normalized distribution, as those more than one standard deviation below the mean, or as below the 20^{th} percentile of an ISI distribution. Qualitatively, extreme events as described herein, are those whose severity (*e.g*., intensity, duration, etc.), and/or frequency exceeds expected and commonly observed outcomes. For example, a patient with long history of convulsions, may develop pulmonary edema after one of these seizures. The fact that its impact (pulmonary edema) is both serious and rare/unprecedented makes this seizure "extreme". It should be noted that for an epileptic event to qualify as extreme it need not only be severe or rare.

A patient may have other certain kinds of seizure events which may be classified as "extreme." Seizure events such as status epilepticus (or increased risk thereof), risk of death, seizure events of a certain energy, severity and/or occurring within certain time intervals, seizures with certain effects (e.g., falls, cardiac and/or respiratory dysfunction, cardiac and/or respiratory distress, etc.), and/or the like, may all be considered extreme seizure events for certain patients. Classifying a seizure event as "extreme" may be based upon the condition of the patient's disease state (e.g., a patient's epileptic disease state), or such a classification may be made in some cases based upon characteristics of the seizure event. In different cases, extreme seizure events may be classified according to other standards as well, and need not necessarily be specifically limited to those described herein. Similarly, extreme seizure events may be a combination of the above described classifications. An extreme seizure event (e.g., status epilepticus or risk of status epilepticus) may result in a pathophysiological effect in a patient such as, but not limited to, damage to brain tissue resulting in permanent and/or serious impairment of motor, visual, sensory and/or cognitive skills, respiratory failure, cardiac failure, pulmonary edema, cardiac arrhythmia, metabolic acidosis, liver and/or renal failure, bed sores, bone fractures, abrasions, bruises, organ or multi-organ failure, arterial hypertension, tissue hypoxia and/or tissue hypercarbia or death.

As described herein, "extreme seizures" may be classified based on certain metrics such as, for example, a seizure severity index (SSI) and/or inter-seizure intervals (ISI) on the impact a seizure has on a subject, *i.e.,* the patient impact (PSimp) or patient seizure burden. Seizure metrics may be: a) peak energy (described for example as the product of peak intensity and duration); b) severity (described for example as the sum of peak energies at each brain site engaged in seizure activity); and/or c) inter-seizure interval (described as the time (in seconds or minutes) elapsed between the onset of consecutive seizures) (see Osorio et al., Epilepsia 1998; 2002; EJN, 2009; PRE 2010).

As indicated above, an impact that seizures may have on a patient may be quantified using various metrics described herein and may be used to determine if a seizure is "extreme". For example, a partial seizure may result in a fall during which the patient fractures a leg bone. The occurrence of a fracture, even though this seizure's SSI and ISI values would not qualify it as extreme, may be used to declare the partial seizure as an extreme epileptic event. Additionally, the frequency and/or ISI(s) of generalized and partial seizures may indicate an extreme epileptic event or state. The examples of generalized and partial seizure extreme epileptic events are exemplary in nature, and the described examples are not exclusive or limiting.

Seizure metrics may include energy, described for example as the product of peak intensity and duration, or as the product of the logarithm of the standard deviation of the difference of signals (*e.g.,* EKG) and the duration. If *Z*(*t*) is the reference EKG signal then its difference from a test epoch will be *X*(*t*) = *Z*(*t*) - *Z*(*t*-1).

These seizure metrics may be derived not only from cortical electrical activity, to date the only signal used for this purpose, but from kinetic (*e.g*., movement, acceleration or force of the body or body parts), cognitive (*e.g.,* awareness, memory), cardiac (*e.g.,* heart rate or heart rate variability), respiratory (*e.g.,* rate, tidal volume, oxygen saturation), metabolic (*e.g.,* pH), tissue stress markers (*e.g.,* lactic acid) or endocrine activity (*e.g.,* cortisol) which provide valuable and reliable information about seizures such that they may be used in lieu of or in addition to electrical cortical activity. For example, the product of peak heart rate and the time it spends above values not observed during seizures may be used to estimate seizure severity.

Severity may also be a seizure metric from which a seizure severity index (SSI) may be determined. The SSI may be observable/measurable data associated with a seizure that is quantifiable. Severity may be described for example as the average of the sum of percentile intensity, duration and extent of spread of changes caused in the brain and other body organs, or as the sum of energies at each brain site engaged in seizure activity or at each organ affected by said seizure activity. According to the invention, the SSI is calculated as the product of the peak intensity of the seizure event and the duration of the seizure event.

The peak intensity may be the maximum value of any one, or any number, of body data values during a seizure event. For example, heart rate, an autonomic index, may be used to compute an SSI as follows: a) As an exemplary illustration, if a patient's mean inter-ictal (in-between seizures) heart rate is 80 beats/minute, the peak ictal heart rate is 150 beats/minute and the increase in heart rate above inter-ictal values lasts for 40 seconds, the SSI is either 6000 if the peak heart rate is used or 2800 if instead the net increase is taken into account. As a further exemplary illustration, the "area under the curve" may be also utilized to compute the SSI; b) If a patient's mean inter-ictal oxygen saturation during wakefulness is 98% and during a convulsion it drops to a minimum of 60%, remaining below the inter-ictal baseline for 60 seconds, the SSI based on this index, is 60 x 60 = 3600, or 33 x 60 = 1980 if instead the net decrease is used. In this example, an SSI above a pre-determined (or adjustable) value may indicate a risk of an extreme epileptic event/state (e.g., status epilepticus). Similarly, an SSI value above or below a pre-determined (or adjustable) percentile based upon historical patient data may indicate an increased risk of occurrence of an extreme event. For example, if an SSI value for a patient is above the ninetieth percentile of the patient's past SSI values, the patient may be at an increased risk for being in an extreme epileptic state (e.g., status epilepticus). The SSI computations described above are provided for exemplary purposes; other type of computations may also be implemented and remain within the spirit and scope of embodiments disclosed herein.

In one embodiment, an SSI value indicative of the severity of a seizure may be determined based upon a body data as described above. In one embodiment, the determined SSI value may be compared to one or more of a reference value (e.g., inter-ictal or non-seizure value), a non-extreme seizure reference value or to or an extreme reference value that may or may not include a status epilepticus value. The status epilepticus value(s) may be based upon at least one of a past SSI value, a mean SSI value, a median SSI value, a mode SSI value, a percentile SSI value, a normalized SSI value, a distribution of SSI values, or to any other statistical transformation of an SE index or observable SE index change. Another useful measure for seizure time series is a post-ictal severity index (PISI) which may be described as the intensity, duration and/or extent of spread (and changes therein) during the post-ictal state, compared to the inter-ictal or to the ictal state.

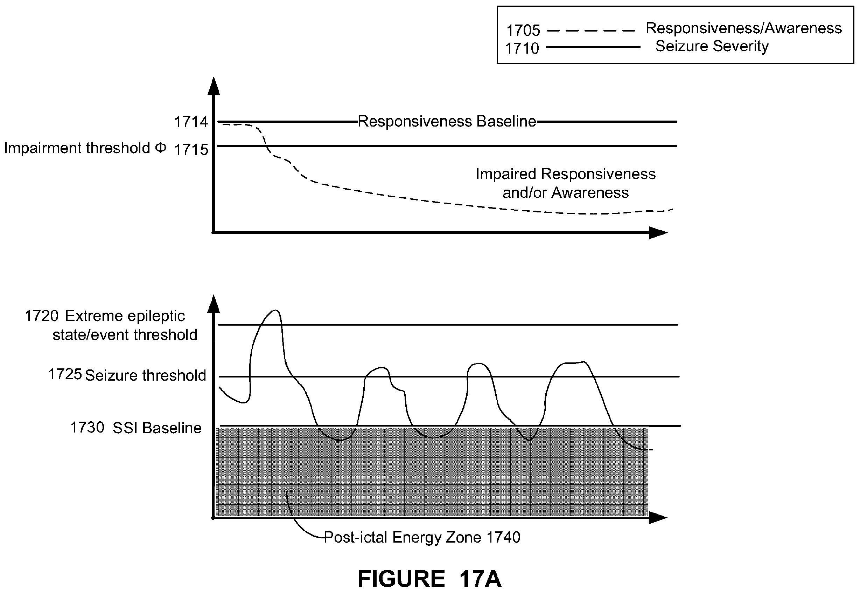

Inter-seizure interval (ISI) may also be a seizure metric, and may be described as the time (in any unit of time) elapsed between the onset of consecutive or non-consecutive seizures, or between the end of a seizure and the start of the next one. In one embodiment, inter-seizure intervals (ISIs) indicative of the time elapsed for example, between the end time and onset time of seizures, may be determined based at least upon body data and by performing statistical analyses to obtain measures of central tendency (*e.g*., mean) after appropriate statistical transformations if indicated, distributions either temporal, spatial or both, and the like. It should be noted that depending upon how the ISI is described, seizure activity may be contained within this interval. Specifically, if the ISI is described as the time from the onset of a seizure to the onset of the next seizure, seizure activity is contained therein (*see, e.g.,* inter-seizure intervals 1790, 1796 and 1798 of Fig. 17A below; each contain seizure activity). The determined ISI value(s) may be compared to non-extreme reference or to an extreme reference value(s). The ISI values may be obtained through direct recording of neuronal/neuropil activity from any potential or known epileptogenic brain site or indirectly through the recording of body signals (*e.g*., other neurologic, autonomic, metabolic, endocrine, tissue stress markers, etc.) that are under control of the potentially epileptogenic brain regions. In one embodiment, the duration of ISIs may be determined for each patient. Further, reference ISI values for a patient, groups or types of patients or types of seizures or of epilepsies, may be determined. These groups may include patients by gender, by age or range of ages, by level of physical fitness/body integrity, by the environment and conditions of said environment, by circadian or ultradian cycles, by level of consciousness, by type and level of cognitive or physical activity, by type of treatment(s) for seizures or other disorders etc. In one embodiment, a database for storing various categories of ISI values may be provided. This database may be used, for example, by the medical device (MD) 200 to perform the assessment of extreme seizure events described herein.

Classification of seizures as extreme events may be based on a) ISI duration that is, for example, one standard deviation below the mean of ISI values or below the 10^{th} percentile of ISI values; or b) the occurrence of seizure before at least one of a plurality of body signal indices has recovered to its inter-ictal value.

Extreme ISIs may increase one or more of SSIs, seizure impact and/or seizure burden, because of the cumulative negative effect on one or more of the body signals used for their detection and quantification. For example, extreme ISIs may result in higher heart rates, more cognitive dysfunction, higher cortisol levels, more profound metabolic acidosis, and/or the like, as well as longer recovery (*e.g*., to baseline levels) than non-extreme ISIs.

In one embodiment, ISI values may be based upon at least one of a time between the onset of a first seizure and the onset of the next seizure, a time between the beginning of the post-ictal period of a first seizure and the onset of the next seizure, a time between the end of the post-ictal period of a first seizure and the onset of the next seizure, a time between the beginning of the post-ictal period of a first seizure and the beginning of the post-ictal period of the next seizure, or a time between the beginning of the post-ictal period of a first seizure and the end of the post-ictal period of the next seizure.

A seizure may be considered extreme (independent of its SSI or ISI or spread values), if it causes system dysfunction of a type, magnitude, duration and/or frequency exceeding the ictal or post-ictal baseline dysfunction for that subject, or if the seizure adversely affects the subject's physical (including the neurologic system) integrity.

The concept of "extreme" may take different meanings for different fields. Extreme value theory in math is a specific corpus in which limit theorems have been developed for the extreme of maximum value of a set of N variables. The term "extreme" as used herein may or may not have this mathematical connotation.

Classifying a seizure event as "extreme" may also be based upon a deleterious impact upon (or seriousness in relation to) the patient's health (*e.g.,* falls, bone fractures, cardiac and/or respiratory dysfunction, memory loss, etc.), and well being or the condition of the patient's disease state (*e.g*., worsening of epilepsy). In different cases, extreme seizure events may be classified according to other standards as well, and need not necessarily be specifically limited to those described herein. Similarly, extreme seizure events may be a combination of one or more of the above described classifications. An extreme seizure state may result in coma, cardio-respiratory failure, metabolic acidosis, liver and/or renal failure, bed sores, bone fractures, tissue hypoxia and brain damage. In one embodiment, an extreme epileptic state is described as two or more extreme events occurring in close temporal proximity to each other.

In one embodiment, determining whether an event is extreme may be based, at least in part, upon: a) an ISI value being shorter than a reference ISI value; b) an occurrence of a first seizure followed by a second seizure, wherein the second seizure occurs prior at least one of an autonomic, neurologic, endocrine, metabolic, tissue stress marker or physical fitness/body integrity returning to inter-ictal values, but being independent of the ISI value. In addition to examining the ISI value, SSI or other index values may also be used to determine whether an extreme event has occurred.

Those skilled in the art having the benefit of this disclosure will appreciate that non-Gaussian distributions may be normalized by, for example, applying to the data logarithmic transformations so that mean, standard deviation and other measures may be more easily estimated. The approach of treating certain seizures as extreme events lends itself to a statistical or probabilistic approach for the prevention of "extreme" seizure events (e.g., status epilepticus) through their anticipation or early detection.

The following list of "metrics," are described in one or more of United States patent applications 13/040,996, 13/091,033, 13/333,235, which are incorporated by reference herein in their entirety, expressed as indices, alone or in any combination, may be used to classify seizures into extreme as compared to non-extreme:
1. Magnitude and/or rate of increase in seizure energy or intensity, duration or extent of spread (referred to herein as the seizure severity index (SSI)); 2. Magnitude and/or rate and/or duration of increase/decrease and of extent of spread, of changes in body indices after the end of a seizure compared to inter-ictal or ictal values, referred herein to as a post-ictal severity index (PISI); 3. Magnitude and/or rate and/or extent of recovery from the post-ictal to the inter-ictal state of any body index; 4. Inter-seizure interval duration; 5. Seizure frequency per unit time, and/or cumulative seizure severity index (SSI) per unit time; 6. Cumulative post-ictal severity index per unit time compared to inter-ictal or ictal index values 7. Magnitude and/or duration and rate of change in level of consciousness; 8. Magnitude, duration and/or rate of changes in one or more cognitive functions; 9. Magnitude and/or duration and/or extent of spread in brain energy; 10. Magnitude, duration and/or rate of changes in autonomic indices; 11. Magnitude, duration and/or rate of changes in metabolic indices; 12. Magnitude, duration and/or rate of changes in endocrine indices; and 13. Magnitude, duration and/or rate of change in tissue stress markers. Comparisons of the above listed metrics and of all the indices listed below, may be made by taking into account time of day, level of consciousness of the patient (*e.g*., awake or asleep), level of physical activity (*e.g*., resting versus moving about) at the time the measurements were made, age, gender and physical fitness/health status or physical integrity.

In one embodiment, a body index may refer to one or more of an autonomic index, a neurologic index, a metabolic index, an endocrine index, a tissue stress index, a physical fitness/body integrity index, and/or a quality of life index. One or more of these body indices may be based upon body data, such as data relating to an autonomic signal, a neurologic signal, a metabolic signal, an endocrine signal, a tissue stress signal, a physical fitness/body integrity signal, or a quality of life signal. The body index values may refer to values computed from one or more body signals detected by one or more sensors operatively coupled (*e.g*., implanted within, in contact with, or wirelessly coupled to the patient's body). Further, a morphology of the body signal indices may be used determine an amount of deviation of the body indices under from predetermined reference values. That is, the shape of the distribution of the body index may be analyzed and compared to reference values to identify one or more of a pathological or non-pathological patient state.

In one or more embodiments, index values indicative of the function the autonomic, neurologic, endocrine, metabolic, gastro-intestinal, and/or of tissue/organ stress, such as those listed in United States patent applications 13/040,996, 13/091,033, 13/333,235, which are incorporated by reference herein in their entirety, along with processes and tools for measuring and/or deriving these signals and markers, may be used to determine the occurrence of seizure events and to classify them as either non-extreme or extreme.

### I. Autonomic:

a) Cardiac: Intra-cardiac pressure, cardiac volume, the ratio of intra-cardiac pressure to cardiac volume, ejection fraction, blood flow, cardiac wall temperature, heart rate variability (HRV), rate of change of HRV as a function of heart rate, heart sounds, heart rhythm, heartbeat wave morphology, point of maximum impulse force, thoracic wall deflection as measured with suitable tools such as EKG, phonocardiogram (PKG), Echocardiography, Apexcardiography (ApKG), and/or the like;
b) Vascular: arterial and or venous pressure, arterial and/or venous blood wave pressure morphology, arterial and/or venous blood flow velocity, arterial and/or venous blood flow sounds, arterial and/or venous temperature as measured with suitable tools such as pressure, Doppler, sound, ultrasound and/or the like;
c) Respiratory: tidal volume, minute volume, respiratory wave morphology, respiratory sounds, intercostal electromyography (EMG), diaphragmatic EMG, at least one chest wall and/or abdominal wall motion, respiratory rate (RR), rate of change of RR, arterial gases concentrations, oxygen saturation, end-tidal CO₂, as measured with suitable tools;
d) Dermal: skin resistance, skin temperature, skin blood flow, sweat gland activity as measured with suitable tools;
e) Neurotransmitters: concentrations of catecholamines and/or catecholamine metabolites, acetylcholine and/or acetylcholinesterase activity in body fluids or other tissues with suitable assays; rate of change of catecholamines, acetylcholine and/or acetylcholinesterase activity as measured from body fluids or other tissues with suitable assays;

### II. Neurologic

a) Cognitive/Behavioral: level of consciousness, level of attention, reaction time, memory, visuo-spatial, language, reasoning, judgment, calculations, auditory and/or visual discrimination as measured using validated tests;
b) Kinetic: force of muscle contraction, body movement direction, speed, acceleration, trajectory of movements in one, two and/or three dimensions, pattern and/or quality of movements, head, eyes, limb/body posture and/or position, body part orientation and/or position in reference to each other, body part orientation and/or position in reference to one or more predetermined axes or fiducials (*e.g*., body incline), muscle tone, agonist-to-antagonist muscle tone ratio, gait, accessory movements, falls as measured with suitable tools;
c) Vocalizations: formed and/or unformed vocalizations as measured with suitable tools;
d) Electroencephalography (EEG), Electrocorticography (ECoG), evoked potentials, field potentials, single unit activity as measured with suitable tools;

### III. Endocrine:

a. prolactin, luteinizing hormone, follicle stimulation hormone, growth hormone, adreno-corticotropin hormone (ACTH), cortisol, vasopressin, beta-endorphin, beta, lipotropin, corticotropin-releasing factor (CRF) as measured from body fluids or other tissues;

### IV. Tissue stress markers:

a. reactive oxygen and/or nitrogen species;

### V. Metabolic:

a. arterial pH, arterial gases, lactate/pyruvate ratio, electrolytes and glucose as measured from body fluids or other tissues.

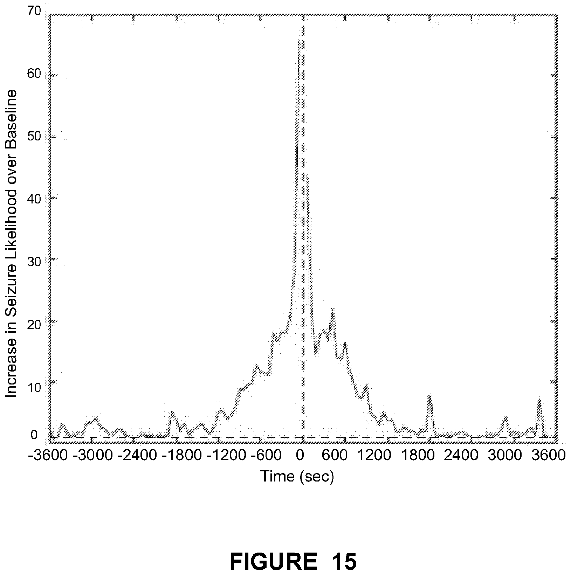

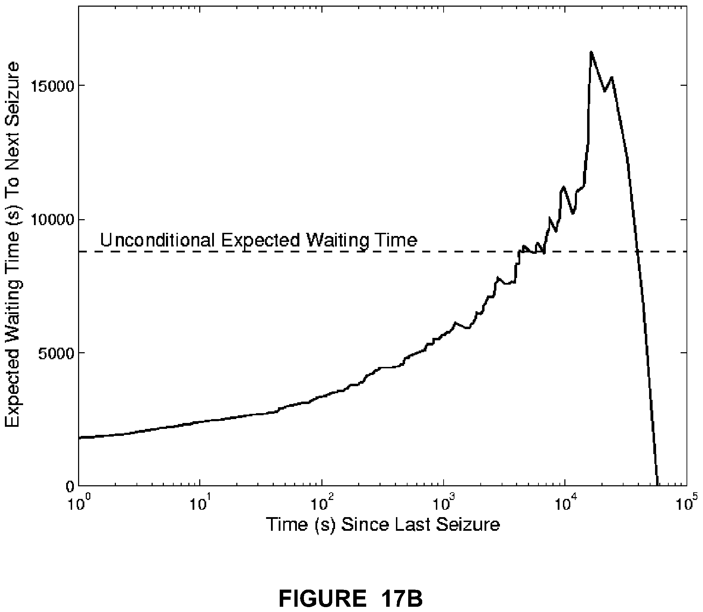

The increased probability of subclinical and clinical pharmaco-resistant seizures to occur in clusters (see Figure 15), an observation previously made for clinical seizures only, and the decreased probability of seizure occurrence with increasing time from the last one (*see e.g.,* Figure 17B) may be interpreted as: (i) reflecting the inherent capacity of seizures to trigger seizures; (ii) indicative of some form of seizure interdependency or plasticity ('memory') in the system, as recently proposed; and/or (iii) a clinically useful observation that in the embodiments herein may be exploited to anticipate and prevent extreme epileptic events including but not limited to status epilepticus.

In one more embodiments, the method may comprise either anticipating and preventing or identifying and/or managing an occurrence of an extreme epileptic event/state both with a certain probability, based upon a comparison of the determined SSI value to a reference or upon models based among other factors on the temporal evolution of SSI, patient seizure impact (PSimp) or ISI values or both. In one embodiment, the impact of the seizure is measured not only on each of the organ/systems of the body, but on the entire body as well.

Although not so limited, methods and apparatus capable of implementing embodiments of the present invention are described below. In the context of this description, a medical device (MD) or medical system may also be referred to as an implantable medical device and/or an implantable medical device/system (IMD). It is contemplated that such a device and/or system may be implantable or non-implantable/non-implanted in various embodiments without departing from the spirit and scope of the invention. In other embodiments, some portions of the system may be implanted while other portions may be external to the patient's body.

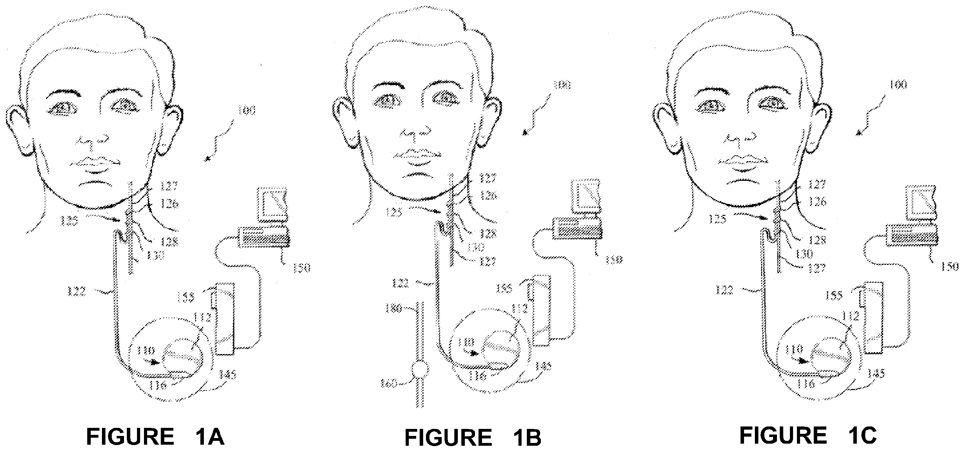

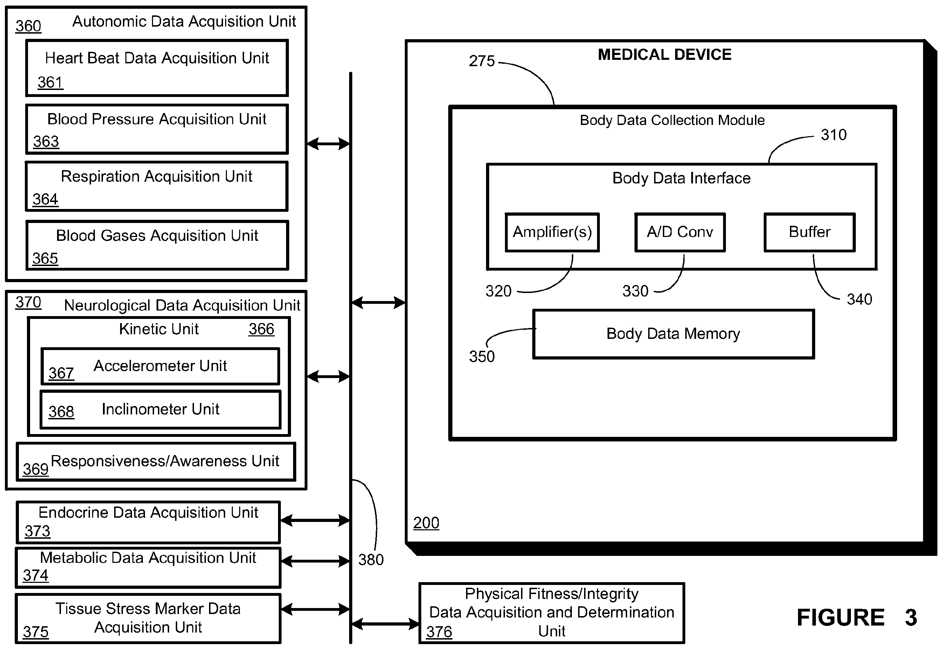

Turning now to Figures 1A-1C, stylized medical systems (MDs) 100 for implementing one or more embodiments of the present invention are depicted. These drawings and MDs 100 are described in A Systems Approach to Disease State and Health Assessment (Figs. 3A-3C) by Dr. Ivan Osorio (U.S. Application Number 12/816,357), incorporated by reference herein in its entirety. It is noted that the described MDs 100 may similarly be implantable/implanted or non-implantable/non-implanted without departing from the spirit and scope of embodiments described herein.

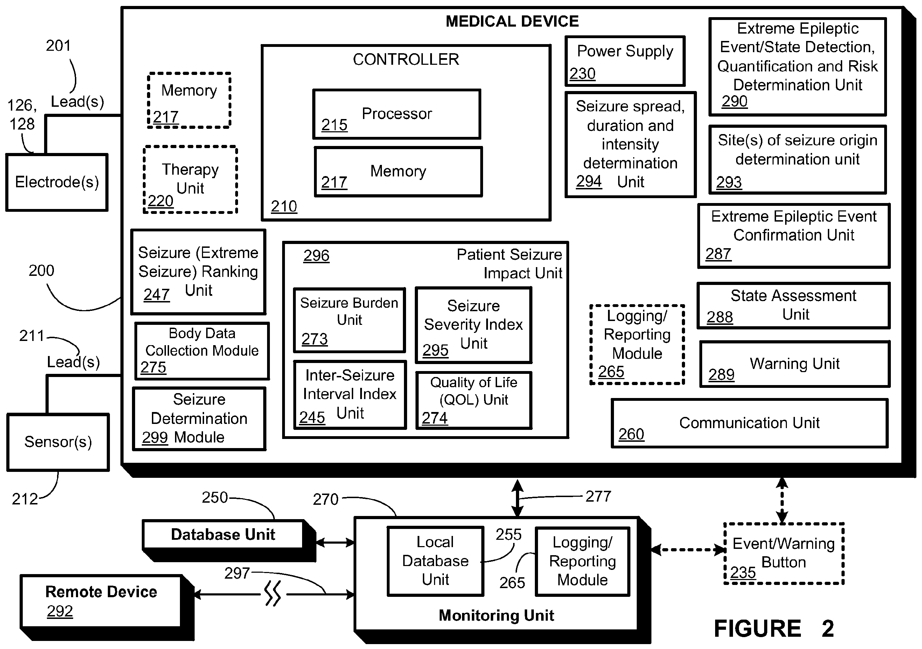

Turning now to Figure 2, a block diagram depiction of a medical device (MD) 200 is provided, in accordance with one illustrative embodiment of the present invention. In some embodiments, the MD 200 may be implantable (such as implantable electrical signal generator 110 from Figure 1), while in other embodiments the MD 200 may be completely external to the body of the patient.

The MD 200 (such as generator 110 from Figure 1) may comprise a controller 210 capable of controlling various aspects of the operation of the MD 200. The controller 210 may include a processor 215, a memory 217, etc., for processing and storing data respectively. The processor 215 may comprise one or more microcontrollers, microprocessors, etc., capable of performing various executions of software and/or firmware components. The memory 217 may comprise various memory portions where a number of types of data (*e.g*., internal data, external data instructions, software codes, status data, diagnostic data, etc.) may be stored. The memory 217 may comprise one or more of random access memory (RAM), dynamic random access memory (DRAM), electrically erasable programmable read-only memory (EEPROM), flash memory, etc. In one embodiment, a memory 217 may be separate from, but communicatively coupled to the controller 210.

The controller 210 is capable of receiving internal data or external data, and in one embodiment, is capable of processing body data to identify an extreme epileptic event in a patient. For example, the controller 210 may receive data from a body data collection module 275 (described further below) or from a memory 217. Upon receiving the data or body data, the processor 215 may process the data in accordance with various embodiments described herein. For example, in one embodiment, the process may be adapted to compare values associated with two or more body data indices. The processor 215 may also provide processed data/body data to other modules and units in the MD 200. The controller 210 is capable of causing a therapy unit 220 to take responsive action in response to the identification of one or more various extreme or non-extreme epileptic events by the MD 200, or by a patient, a physician, a nurse or caregiver, etc. In one embodiment, the responsive action may comprise generating and delivering an electrical signal to target tissues of the patient's body for treating a medical condition. In one or more embodiments, the responsive action may comprise drug treatments, oxygen treatments, cooling and/or the like. For example, the controller 210 may receive manual instructions from an operator externally, or may cause the electrical signal to be generated and delivered based on internal calculations and programming. In other embodiments, the MD 200 does not comprise a therapy unit 220. In either embodiment the controller 210 is capable of affecting, and/or may be adapted to affect, substantially all functions of the MD 200.

As stated above, in one embodiment, the MD 200 may also comprise a therapy unit 220 capable of generating and delivering electrical signals to one or more electrodes 126, 128 via leads 201 (Figures 2B, 2D) (and/or other therapies such as drugs, thermal energy, oxygen and/or the like). A lead assembly such as lead assembly 122 (Figure 1) may be coupled to the MD 200. Therapy may be delivered through the leads 201 comprising the lead assembly 122 by the therapy unit 220 based upon instructions from the controller 210. The therapy unit 220 may comprise various circuitries, such as electrical signal generators, impedance control circuitry to control the impedance "seen" by the leads (*i.e.,* the impedance at the leads), and other circuitry that receives instructions relating to the delivery of the electrical signal to tissue. Electrical signals delivered to a body part for therapeutic purposes may be of constant current (to compensate for impedance changes) or of constant voltage. The therapy unit 220 is capable of delivering electrical signals over the leads 201 comprising the lead assembly 122. As should be apparent, in certain embodiments, the MD 200 does not comprise a therapy unit 220, lead assembly 122, or leads 201. In particular, although Figures 2B and 2D are illustrated with therapy unit 220, leads 201 and electrodes 126, 128, in alternative embodiments, these structures and the stimulation function enabled thereby may be omitted.

In other embodiments, a lead 201 is operatively coupled to an electrode 126, 128, wherein the electrode 126, 128 is adapted to couple to at least one of a portion of a brain structure of the patient 190, a cranial nerve of a patient, a spinal cord of a patient 180, a sympathetic nerve structure of the patient, a peripheral nerve of the patient, a dermis and/or subdermis of a patient.

The MD 200 may also comprise a power supply 230. The power supply 230 may comprise a battery, voltage regulators, capacitors, etc., to provide power for the operation of the MD 200, including delivering the therapeutic electrical signal. The power supply 230 comprises a power source that in some embodiments may be rechargeable. In other embodiments, a non-rechargeable power source may be used. The power supply 230 provides power for the operation of the MD 200, including electronic operations and the electrical signal generation and delivery functions. The power supply 230 may comprise a lithium/thionyl chloride cell or a lithium/carbon monofluoride (LiCFx) cell if the MD 200 is implantable, or may comprise conventional clock, watch or 9V batteries for external (*i.e.,* non-implantable) embodiments. Other battery types known in the art may also be used.

The MD 200 may also comprise a communication unit 260 capable of facilitating communications between the MD 200 and various devices. In particular, the communication unit 260 is capable of providing transmission and reception of electronic signals to and from a monitoring unit 270, such as a handheld computer or PDA that can communicate with the MD 200 wirelessly or by cable. The communication unit 260 may include hardware, software, firmware, or any combination thereof.

The MD 200 may also comprise one or more sensor(s) 212 coupled via sensor lead(s) 211 to the MD 200. The sensor(s) 212 are capable of receiving signals related to a body parameter, such as the patient's heart beat or a body chemical, and delivering the signals to the MD 200. In one embodiment, the sensor(s) 212 may be the same as implanted electrode(s) 126, 128 (Figure 1). In other embodiments, the sensor(s) 212 are separate structures that may be placed on the patient's skin, such as over the patient's heart or elsewhere on the patient's body. It will be appreciated by persons of skill in the art that in some embodiments, lead 211 may be omitted and the MD 200 may communicate wirelessly with sensor 212.

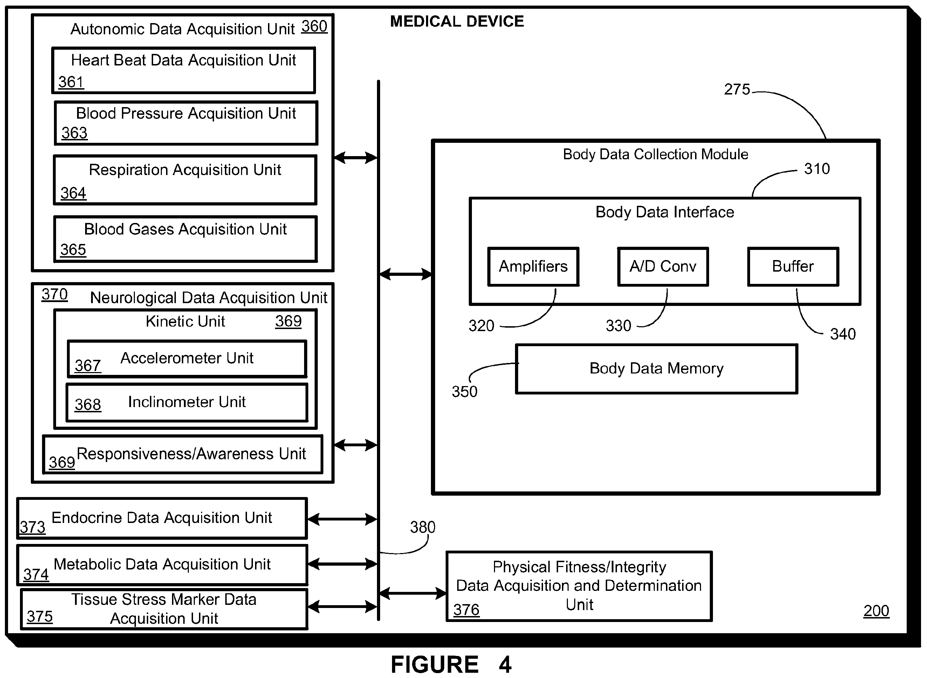

The MD 200 may also comprise a body data collection module 275. The body data collection module 275 may be adapted to, and/or capable of, collecting data relating to the body of a patient. Such data may be obtained using electrical, chemical, optical, biophotonic, and/or acoustic (*e.g*., ultrasound) signals/indicators, thermal sensors, pressure sensors, bioassays, chemical methods, imaging technology and/or motion sensors in any useful combination (these measurements may be performed at one or multiple spatial scales simultaneously or sequentially (*e.g*., multiplexing) and include but are not limited to: 1. Neurologic data; 2. Cardiac signals; 3. Body fluids signals; 4. Respiratory signals/indices; 5. Endocrine indices; 6. Metabolic parameters; and 7. Kinetic data. A more complete, but not exclusive list of body data examples may be found in U.S. patent applications 13/040,996, 13/091,033, 13/333,235, which are incorporated by reference herein in their entirety, and the body data collection module 275 may collect additional data not listed herein, that would become apparent to one of skill in the art having the benefit of this disclosure. The body data collection module 275 may collect body data via one or more body data units (described in further detail below with respect to exemplary embodiments shown in Figs. 3 & 4, [361-368]). The body data units, as shown below, may be internal to the MD 200, or external to, and communicatively coupled to, the MD 200. All data comparisons may be made taken into consideration including, but not limited to, ultradian, circadian or infradian variations, gender, body mass index, age, past and present treatments (*e.g*., drugs, electrical signal therapy, and/or the like), physical fitness/integrity, etc.

The body data collection module 275 may, in some embodiments, organize or process portions of the body data collected. Additionally, the body data collection module 275 may store or buffer the body data before sending the body data to other components of the MD 200. In accordance with one embodiment, the body data collection module 275 may send some or all of the body data collected by the body data collection module 275 to the controller 210 for processing. In other embodiments, the body data collection module 275 may send collected body data to other components of the medical device instead of, or in addition to, the controller 210; such other components include, but are not limited to, a SUDEP risk determination unit 285, a seizure severity ranking unit 299, a seizure severity index (SSI) unit 295 and an extreme epileptic event/state detection, quantification and risk determination unit 290. The body data collection module 275 may also send the body data to the monitoring unit 270 or remote device(s) 292 via communication unit 260.

The MD 200 may also comprise a seizure spread, duration and intensity determination unit 294. In accordance with one embodiment, the seizure spread, duration and intensity determination unit 294 may determine the amount of spread the seizure event and/or extreme seizure event.

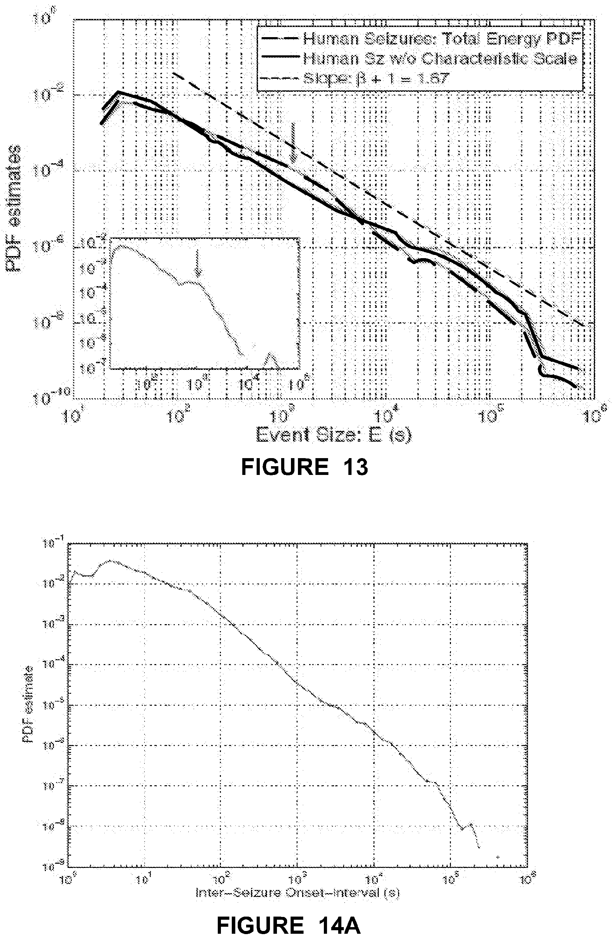

The seizure spread, duration and intensity determination unit 294 may be adapted to provide seizure duration data which may include the duration of a patient's seizure event. The duration of a seizure event may be determined as the time that any of the autonomic, neurologic, metabolic, endocrine, or stress tissue marker indices values differ from inter-ictal and/or post-ictal values. In some embodiments, electrographic or clinical onset may be approximated by other body parameters, e.g., heart rate, kinetic activity, etc. In alternative embodiments, the duration of a seizure event may be determined as the time a body data value, a site(s) data value and/or an intensity, duration and spread data value (or value(s) respectively related thereto) is above or below adaptable and/or pre-determined threshold(s) and/or separatrix(tices). In further embodiments, the duration of a seizure event may be based on other criteria as would be apparent to one of skill in the art having the benefit of this disclosure. The seizure spread, duration and intensity determination unit 294 may also be adapted to determine a time spent in a seizure event state over a given time period or window (*e.g*., macroscopic as described above); such a determination may include one or more seizure events occurring within the time period (as discussed in further detail below with respect to Figure 13). The time periods/windows may be of a fixed duration or may be of adjustable duration; likewise, the time period or window may move with or without overlap.

The seizure spread, duration and intensity determination unit 294 may be adapted to provide seizure intensity data. As described above, seizure intensity may be described as the value of any one, or any number, of body data values during a seizure event. A maximum intensity of a seizure may be described as the maximum value of any one, or any number, of body data values during a seizure event (*e.g.,* the maximum heart rate of a patient during a seizure event).

The MD 200 may also comprise a seizure severity index (SSI) unit 295. SSIs may be calculated in some embodiments using at least two of seizure intensity, duration or extent of spread and using at least of one autonomic, endocrine, metabolic, stress tissue marker signals. For example, SSI may be the product of intensity and spread or the sum of intensity, duration and spread using one or more organ/system indices. In accordance with one embodiment, the SSI unit 295 may determine one or more seizure severity indices (SSIs) based upon the body data collected by the body data collection module 275 and/or other relevant data. An SSI may be a scalar-valued function of one or more body data variables that simplifies a possibly complex set of body information down to a single number: the SSI. In accordance with one embodiment, the SSI may be any statistic (or scalar-valued function) associated with a seizure with the property values that reflect some aspect of the severity of the corresponding seizures and may be ordered/sorted so that the distance between the SSI values for different seizures can be measured, compared and/or interpreted to provide meaningful information. In one embodiment, the SSI may be a quantity whose variation over a period of time measures the change in some body data or body phenomenon. In one embodiment, the SSI may be intended to generally reflect the impact of a seizure on a body organ or system. The SSI may also be a statistic associated with the seizure that enables comparison between different seizures, and the values for different seizures may be ordered/sorted and the distance (in a Euclidian or non-Euclidian sense) between them measured/compared/interpreted to provide meaningful information. If the SSI values describe the severity of the seizure not in absolute terms, but in a manner relative to other seizures for that patient (or relative to other patients), the SSI may be referred to as a "Relative SSI." Additionally, when more than one SSI is used at the same time, the plurality of SSIs may be combined into a single SSI by weighted averaging, and/or the like.

The MD 200 may also comprise an inter-seizure interval index unit 245. In accordance with one embodiment, the inter-seizure interval index unit 245 may determine an index based upon inter-seizure interval. The inter-seizure interval index may, in some embodiments, be representative of the current inter-seizure interval relative to past single values, sets or values or value distributions and/or expected inter-seizure intervals, either for a specific patient or for one or more patients or patient populations. The inter-seizure interval index unit 245 may make such rankings based upon body data information, external indications (e.g., the patient's environment or surroundings), a patient's past seizure data, a normalized seizure data distribution, expected seizure data and/or other data that would become apparent to one of skill in the art having the benefit of this disclosure. Additionally, the inter-seizure interval index unit 245 may base its index upon a comparison of any or all of the above referenced data, information or indications.

Inter seizure intervals (ISIs), post-ictal severity indices (PISIs), seizure severity indices (SSIs) and patient seizure impact (PSimp) may be used alone or in combination to determine the probability that an extreme event will occur, is occurring or has occurred. These data may be derived using one or more autonomic (*e.g.,* cardiac, respiratory, dermal), endocrine (*e.g.,* prolactin), metabolic (*e.g.,* arterial pH), tissue stress markers (*e.g.,* CK) or neurologic (*e.g.,* kinetic, cognitive) signals.

In one more embodiments identifying an occurrence of an extreme event with a certain probability or an increased risk of an extreme event state with a certain probability may be based upon a comparison of a determined SSI, ISI or PSimp values or upon models based, among others, on the temporal evolution of SSI, ISI or PSimp (for a specific patient or patient populations) in any combination.

The MD 200 may also comprise a seizure (extreme seizure) ranking unit 247. In accordance with one embodiment, the seizure (extreme seizure) ranking unit 247 may determine a ranking of a seizure event and/or extreme seizure based upon severity (*e.g*., SSI), ISI, PISI, PSimp, PSB and/or other factors. In one or more embodiments, the seizure (extreme seizure) ranking unit 247 may rank either seizure events or extreme seizure events. That is, in various embodiments, the seizure (extreme seizure) ranking unit 247 may be used for ranking one of seizure events or extreme seizure events. Alternatively, in one embodiment, multiple instances of the seizure (extreme seizure) ranking unit 247 may be implemented in the MD 200 (*e.g.,* one instance for ranking seizure events and one instance for ranking extreme seizure events). As such, specific determinations relative to seizure events and/or extreme seizure events may be made independently of each other. For instance, a library or report log of just extreme seizure events may be maintained; this may allow for extreme seizure event comparisons and rankings which need not include non-extreme seizure events.

The ranking of seizure events and/or extreme seizure events may, in some embodiments, be based upon a reference value that may in turn be based upon normative, reference and/or historical patient data, or the like, which may be patient-specific or for particular patient populations. In the case of extreme events, an extreme reference value may be used. An extreme reference value may be a reference value above and beyond that used to indicate non-extreme seizure events. In other words, the seizure (extreme seizure) ranking unit 247 may make such rankings based upon body data information, external indications, a patient's past seizure data and/or other data that would become apparent to one of skill in the art having the benefit of this disclosure. Additionally, the seizure (extreme seizure) ranking unit 247 may base its ranking(s) upon a comparison of any or all of the above referenced data, information or indications.

The MD 200 may also comprise a site(s) of seizure origin determination unit 293. In accordance with one embodiment, the site(s) of seizure origin determination unit 293 may determine the site or sites of origin of a seizure event and/or extreme seizure event in a patient's brain. This information may be used to determine different types of seizure events, rank them according to severity (SSI), ISI, PISI, PSimp and/or PSB according to site of origin and classify them as extreme or non-extreme events. The site(s) of seizure origin determination unit 293 may make such a determination based upon body data information, external indications, and/or other data that would become apparent to one of skill in the art having the benefit of this disclosure. In one embodiment, patients in whom seizures originate from more than one brain site ("focus") within a region, one region in a lobe, one lobe within a hemisphere and/or one hemisphere, SSI, ISI, PISI, PSimp and/or PSB values may be determined from each site, region, lobe and/or hemisphere by performing statistical analyses to obtain measures of central tendency (e.g., mean), distributions (either temporal, spatial or both), and comparing the determined SSI value to reference/extreme reference value(s) that may or may not include a status epilepticus value. The status epilepticus value may be based upon at least one determination of if a status epilepticus event is occurring or the probability that it may occur.

The MD 200 may also comprise a seizure determination module 299. In accordance with one embodiment, the seizure determination module 299 may determine whether or not a patient has had, or is having, a seizure/extreme seizure event using body data. The seizure determination module may make such a determination based upon body data information, external indications, and/or other data that would become apparent to one of skill in the art having the benefit of this disclosure.

The MD 200 may also comprise a patient seizure impact (PSimp) unit 296 which, in one or more embodiments, may comprise a patient seizure burden (PSB) unit 273 adapted to determine a seizure burden on the patient (in another embodiment the PSB unit 273 may be separate from the PSimp unit 296), both of which are more fully described in U.S. patent applications 13/040,996, 13/091,033, 13/333,235, which are incorporated by reference herein in their entirety.

In addition to the PSimp and PSB measures, comparisons of SSI, PISI and/or ISI values to historical values allows quantification of the evolution of epilepsy as a function of time, therapies and preventive measures. For example, by plotting SSI values on the y-axis over a time window (x-axis) trends in the direction of improvement, worsening or stabilization of epilepsy may be easily determined. Increases in SSI or PISI values and/or decreases in ISI as a function of treatment may require a warning and appropriate therapy/treatment.

In another embodiment, a quality of life (QOL) unit 274 (which is more fully described in U.S. patent applications 13/040,996, 13/091,033, 13/333,235, which are incorporated by reference herein in their entirety) may be incorporated into the PSimp unit 296 to determine the impact of epilepsy and seizures on a patient's QOL. The QOL unit 274 may be adapted to determine/quantify one or more QOL factors for a patient, such as mood, sense of well-being (or lack of it thereof), sexual activity and/or the like.

The MD 200 may also comprise an extreme epileptic event/state detection, quantification and risk determination unit 290. In accordance with one embodiment, the extreme epileptic event/state detection, quantification and risk determination unit 290 may determine a current state and/or a future risk of entering a status epilepticus state. In one embodiment, the extreme epileptic event/state detection, quantification and risk determination unit 290 may identify a status epilepticus event from a group consisting of a present status epilepticus state, a past status epilepticus state or an increased risk of a status epilepticus state. The determination and/or identification of a risk/state of an extreme event such as status epilepticus may be made based in part or whole upon a comparison of one or more SSI, PISI and/or ISI values to a status epilepticus threshold value(s) or to a reference/extreme reference value that may in turn be based upon reference, normative and/or historical patient data, or the like.

The MD 200 may also comprise an extreme epileptic event confirmation unit 287. In accordance with one embodiment, the extreme epileptic event confirmation unit 287 may confirm that a patient is having an extreme epileptic event and that said event is status epilepticus. The extreme epileptic event quantification and confirmation unit 287 may also confirm that a patient remains in a state of status epilepticus subsequent to an initial detection/identification of the state of status epilepticus.

The MD 200 may also comprise a state assessment unit 288. In accordance with one embodiment, the state assessment unit 288 may indicate various states of a patient's disease, including but not limited to, a status epilepticus event, a risk of SUDEP, a current seizure event, variations in body data indicative of an event/change of a patient's disease state, and the like. In one embodiment, the indication may be provided to other components within the MD 200, to the monitoring unit 270 and/or database unit 250 and/or local database unit 255, to a remote device 292, to the PSimp unit 296, to the PSB unit 273, to a patient, to a caregiver or physician, or the like. The state assessment unit 288 may further indicate that the state or change in state of the patient's disease should be logged, for example, in database unit 250, local database unit 255, and/or the like.

The MD 200 may also comprise a warning unit 289. In accordance with one embodiment, the warning unit 289 may issue a warning to a patient, physician and/or care giver. Such a warning may be indicative of various states of a patient's disease, including but not limited to, an extreme epileptic event such as status epilepticus, a risk of SUDEP, a current seizure event, variations in body data indicative of an event/change of a patient's disease state, and the like, as described above with respect to the state assessment unit 288. Additionally, the warning unit 289 may warn that an event presents an increased risk to the health and/or safety of the patient. The warning unit 289 may provide a warning for a patient, physician and/or care giver to take some immediate or otherwise urgent action related to the event/change of a patient's disease state. The warning unit 289 may warn in addition, or alternatively, to the indication provided by the state assessment unit 288 described above.

The MD 200 may also comprise an event/warning button 235. In accordance with one embodiment, the event/warning button 235 may be located external to the MD 200 in an implanted/non-implanted embodiment, or may be part of the MD 200 in non-implanted embodiments. The event/warning button 235 may be communicatively coupled to the MD 200 and/or to the monitoring unit 270 in various embodiments. The event/warning button 235 may allow for a patient or other individual (such as a caregiver, family member or emergency response personnel) to activate a warning to identify a seizure event and/or an extreme seizure event/state. Such activation may be used to warn of, treat and/or log a seizure event and/or an extreme seizure event/state. Additionally, in one or more embodiments, the event/warning button 235 may be used to elevate a warning or therapy for an existing seizure event and/or an extreme seizure event/state.

In addition to components of the MD 200 described above, a non-implantable/implantable medical system may comprise a storage unit to store an indication of at least one of epilepsy event (*e.g.,* a seizure or an increased risk of a seizure). The storage unit may be the memory 217 of the MD 200, another storage unit of the MD 200, or an external database, such as the local database unit 255 or a remote database unit 250. The MD 200 may communicate the indication via the communications unit 260. Alternatively or in addition to an external database, the MD 200 may be adapted to communicate the indication to at least one of a patient, a caregiver, or a healthcare provider.

In various embodiments, one or more of the units or modules described above may be located in a monitoring unit 270 or a remote device 292. For example, in one exemplary embodiment, the extreme epileptic event/state detection, quantification and risk determination unit 290 may be external to the MD 200, *e.g.,* in a monitoring unit 270 or a remote device 292. Locating the extreme epileptic event/state detection, quantification and risk determination unit 290 outside the MD 200 may be advantageous if the status epilepticus risk determination or detection parameter calculation is computationally intensive, in order to reduce energy expenditure and heat generation in the MD 200 or to expedite calculation of the at least one status epilepticus risk determination or detection parameter.

The monitoring unit 270 may be a device that is capable of transmitting and receiving data to and from the MD 200. In one embodiment, the monitoring unit 270 is a computer system capable of executing a data-acquisition program. The monitoring unit 270 may be controlled by a healthcare provider, such as a physician, remotely at a base station, for example, from a doctor's office or also directly. In alternative embodiments, the monitoring unit 270 may be controlled by a patient in a system providing less interactive communication with the MD 200 than another monitoring unit 270 controlled by a healthcare provider. Whether controlled by the patient or by a healthcare provider, the monitoring unit 270 may be a computer, preferably a handheld computer or PDA, but may alternatively comprise any other device that is capable of electronic communications and programming, e.g., hand-held computer system, a PC computer system, a laptop computer system, a server, a personal digital assistant (PDA), an Apple- UNIX- or Windows-based computer system, a cellular telephone, etc. The monitoring unit 270 may download various parameters and program software into the MD 200 for programming the operation of the medical device, and may also receive and upload various status conditions and other data from the MD 200. Communications between the monitoring unit 270 and the communication unit 260 in the MD 200 may occur via a wireless or other type of communication, represented generally by line 277 in Figure 2. This may occur using, *e.g.,* a wand 155 to communicate by RF energy with an MD 200. Alternatively, the wand may be omitted in some systems, e.g., systems in which the MD 200 is non-implantable, or implantable systems in which monitoring unit 270 and MD 200 operate in the MICS bandwidths.

Likewise, in various embodiments the remote device 292 may communicate with the monitoring unit 270, and thus with the MD 200, with communications between the remote device 292 and the monitoring unit 270 represented generally by line 297 in Figure 2. Communications between the monitoring unit 270 and the remote device 292 may occur via a wireless or other type of communication represented by line 277.

In one embodiment, the monitoring unit 270 may comprise a local database unit 255. Optionally or alternatively, the monitoring unit 270 may also be coupled to a database unit 250, which may be separate from monitoring unit 270 (*e.g.,* a centralized database wirelessly linked to a handheld monitoring unit 270). The database unit 250 and/or the local database unit 255 are capable of storing various patient data. This data may comprise patient parameter data acquired from a patient's body and/or therapy parameter data. The database unit 250 and/or the local database unit 255 may comprise data for a plurality of patients, and may be organized and stored in a variety of manners, such as in date format, severity of disease format, etc. The database unit 250 and/or the local database unit 255 may be relational databases in one embodiment. A physician may perform various patient management functions (e.g., programming parameters for a responsive therapy and/or setting thresholds for one or more event detection parameters) using the monitoring unit 270, which may include obtaining and/or analyzing data from the MD 200 and/or data from the database unit 250 and/or the local database unit 255. The database unit 250 and/or the local database unit 255 may store various patient data.

The MD 200 may also comprise a logging/reporting module 265. The logging/reporting module 265 may be adapted to log and/or store data related to the patient, the patient's physical condition, the patient's disease and disease state and/or any other body data. The logging/reporting module 265 may be adapted to log and/or store information indicative of events relating to the patient's disease (*e.g*., seizure events, data related to time of recovery after seizure events and/or patient sleep-wake cycles). The logging/reporting module 265 may also be adapted to log and/or store a timestamp indicative of the time and day on which stored data is/was acquired. The logging/reporting module 265 may be adapted to report stored data, or any portion thereof, to a patient, a physician, a care giver, an external computer 150, a database unit 250, a local database unit 255 and/or a remote device 292. It is contemplated that the logging/reporting module 265 may not be present in the MD 200 in various embodiments, or alternatively, that the logging/reporting module 265 may be located in a monitoring unit 270 or a remote device 292.

One or more of the blocks illustrated in the block diagram of the MD 200 in Figure 2, may comprise hardware units, software units, firmware units, or any combination thereof. Additionally, one or more blocks illustrated in Figure 2 may be combined with other blocks, which may represent circuit hardware units, software algorithms, etc. Additionally, any number of the circuitry or software units from the various blocks illustrated in Figure 2 may be combined into a programmable device, such as a field programmable gate array, an ASIC device, etc.

The medical device system, in one exemplary embodiment, provides for software module(s) that are capable of acquiring, storing, and processing various forms of data, such as patient data/parameters (*e.g*., body data such as heart rate, breathing rate, brain-activity parameters, PSimp data, PSB data (*e.g*., disease progression or regression data, quality of life data, etc.), and/or the like) as well as therapy parameter data (*e.g*., adverse effects). Therapy parameters may include, but are not limited to, electrical signal parameters that define therapeutic electrical signals delivered by the medical device in response to the detection of an epilepsy event, medication parameters and/or any other therapeutic treatment parameter. Therapy parameters for a therapeutic electrical signal may also include, but are not limited to, a current amplitude, a pulse width, a pulse shape, a frequency, an on-time, an off-time, etc.

In one exemplary embodiment, at least one electrode may be coupled to each of two or more cranial nerves. (In this context, two or more cranial nerves means two or more nerves having different names or numerical designations, and do not refer to the left and right versions of a particular nerve. However, bilateral (left and right) stimulation of the same nerve may be also carried out). In one embodiment, at least one electrode may be coupled to either or both vagus nerves or a branch of either or both vagus nerves. The term "operatively" coupled may include direct or indirect coupling. Each of the nerves in this embodiment or others involving two or more cranial nerves may be stimulated according to particular activation modalities that may be independent between the two nerves. In another embodiment, a therapy may be delivered directly to the brain.

Turning now to Figure 3, a block diagram depiction of an MD 200 is provided, in accordance with one illustrative embodiment of the present invention. Figure 3 depicts an exemplary implementation of the body data collection module 275 described above with respect to Figure 2. The body data collection module 275 may include hardware (*e.g*., amplifiers, accelerometers), tools for chemical assays, optical measuring tools, a body data memory 350 for storing and/or buffering data in the body data collection module 275. The body data collection module 275 may also include one or more body data interfaces 310. The body data interface 310 may provide an interface for input/output (I/O) communications between the body data collection module 275 and body data units/modules (*e.g*., [360-370], [373-376]) via connection 380. Connection 380 may be a wired or wireless connection, or a combination of the two. The connection 380 may be a bus-like implementation or may include an individual connection (not shown) for each, or some number, of the body data units (*e.g*., [360-370], [373-376]). The connection 380 may also include connection elements as would be known to one of skill in the art having the benefit of this disclosure. In various embodiments, the body data units may include, but are not limited to, an autonomic data acquisition unit 360, a neurologic data acquisition unit 370, and endocrine data acquisition unit 373, a metabolic data acquisition unit 374, a tissue stress marker data acquisition unit 375, a QOL unit 274 and/or a physical fitness/integrity acquisition and determination unit 376. In one embodiment, the autonomic data acquisition unit 360 may include a heart beat data acquisition unit 361 adapted to acquire a phonocardiogram (PKG), EKG, echocardiophraphy, apexcardiography and/or the like, a blood pressure acquisition unit 363, a respiration acquisition unit 364, a blood gases acquisition unit 365, and/or the like. In one embodiment, the neurologic data acquisition unit 370 may contain a kinetic unit 366 that may comprise an accelerometer unit 367, an inclinometer unit 368, and/or the like; the neurologic data acquisition unit 370 may also contain a responsiveness/awareness unit 369 that may be used to determine a patient's responsiveness to testing/stimuli and/or a patient's awareness of their surroundings. The body data units ([360-370], [373-376]) may be adapted to collect, acquire, receive/transmit heart beat data, EKG, PKG, echocardiogram, apexcardiogram, blood pressure, respirations, blood gases, body acceleration data, body inclination data, EEG/ECoG and/or the like.

The body data interface(s) 310 may include various amplifier(s) 320, one or more A/D converters 330 and/or one or more buffers 340 or other memory (not shown). In one embodiment, the amplifier(s) 320 may be adapted to boost and condition incoming and/or outgoing signal strengths for signals such as those to/from any of the body data units/modules (*e.g.,* ([360-370], [373-376])) or signals to/from other units/modules of the MD 200. The A/D converter(s) 330 may be adapted to convert analog input signals from the body data unit(s)/module(s) into a digital signal format for processing by controller 210 (and/or processor 215). A converted signal may also be stored in a buffer(s) 340, a body data memory 350, or some other memory internal to the MD 200 (*e.g*., memory 217, Fig. 2) or external to the MD 200 (*e.g*., monitoring unit 270, local database unit 255, database unit 250, and remote device 292). The buffer(s) 340 may be adapted to buffer and/or store signals received by the body data collection module 275 as well as signals to be transmitted by the body data collection module 275. In various embodiments, the buffer(s) 340 may also be adapted to buffer and/or store signals in the body data collection module 275 as these signals are transmitted between components of the body data collection module 275.

As an illustrative example, in one embodiment, data related to a patient's respiration may be acquired by respiration unit 364 and sent to the MD 200. The body data collection module 275 in the MD 200 may receive the respiration data using body data interface(s) 310. As the data is received by the body data interface(s) 310, the incoming data may be amplified/conditioned by amplifier(s) 320 and then converted by A/D converter(s) into a digital form. The digital signal may be buffered by a buffer(s) 340 before the data signal is transmitted to other components of the body data collection module 275 (*e.g.,* body data memory 350) or other components of the MD 200 (*e.g.,* controller 210, processor 215, memory 217, communication unit 260, seizure determination module 299, SSI unit 295, extreme epileptic event/state detection, quantification and risk determination unit 290, or the like). Body data in analog form may be also used in one or more embodiments.

Turning now to Figure 4, an MD 200 (as described above in Figure 3) is provided, in accordance with one illustrative embodiment of the present invention. Figure 4 depicts the body data units (Figs. 3 & 4, [360-370], [373-376]), in accordance with one embodiment, being externally coupled to the MD 200, instead of being included within the MD 200 as shown in Figure 3. In accordance with various embodiments, any number and type of body data units (Figs 3 & 4, [360-370], [373-376]) may be included within the MD 200, as shown in Figure 4 while other body data units (Figs 3 & 4, [360-370], [373-376]) may be externally coupled, as shown in Figure 3. The body data units (Figs 3 & 4, [360-370], [373-376]) may be coupled to the body data collection module 275 in a fashion similar to that described above with respect to Figure 3 (380), or in any number of different manners used in coupling intra-medical device modules and units.

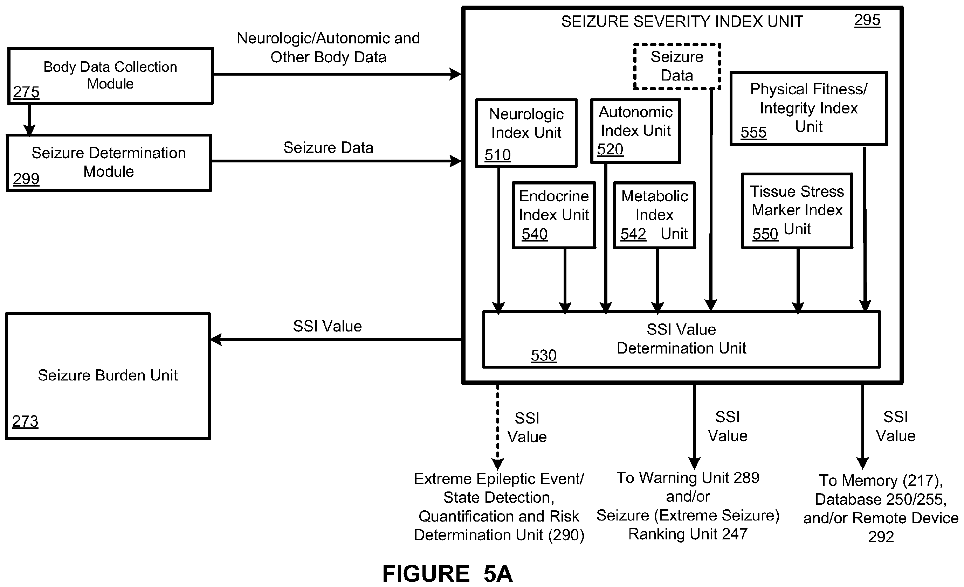

Turning now to Figure 5A, a block diagram depiction of a seizure severity index unit 295 (SSI unit) is provided, in accordance with one illustrative embodiment of the present invention. In one embodiment, the SSI unit 295 may be adapted to determine a seizure severity index (SSI). The SSI unit 295 may use body data and/or seizure data (*e.g*., seizure intensity data, seizure duration data and/or seizure spread data) in determining the SSI. In one embodiment, body data collection module 275 may send body data to SSI unit 295. In one embodiment, the SSI unit 295 may include at least one of a neurologic index unit 510, an autonomic index unit 520, an endocrine index unit 540, a metabolic index unit 542 and/or a stress marker index unit 550 and/or a physical fitness/integrity index unit 555. The neurologic index unit 510 may be adapted to determine a neurologic index value using neurologic body data from the body data collection module 275. The autonomic index unit 520 may be adapted to determine an autonomic index value using autonomic body data from the body data collection module 275. Examples of autonomic indices may include, but are not limited to, heart rate variability heart rate, cardiac cycle morphology, respiratory rate, tidal volume, respiratory cycle morphology, oxygen saturation, and end tidal CO2 saturation, etc. Examples of a cardiac cycle morphology may include, but are not limited to, a PQRST morphology, an ST interval, a QT interval, etc. Examples of a respiratory cycle morphology may include Cheyne-Stokes, ataxic breathing, apneustic breathing, etc.

The endocrine index unit 540 may be adapted to determine an endocrine index value using body data from the body data collection module 275. The metabolic index unit 542 may be adapted to determine a metabolic index value using body data from the body data collection module 275. The stress marker index unit 550 may be adapted to determine a stress marker index value using body data from the body data collection module 275. The physical fitness/integrity index unit 555 may be adapted to determine a stress marker index value using body data from the body data collection module 275. It is noted that the seizure determination module 299 may send seizure data to the SSI unit 295 and that units 510, 520, 540, 542, 550 and 555 may be adapted to determine their respective indices based on seizure data from seizure determination module 299.

The neurologic index unit 510, autonomic index unit 520, endocrine index unit 540, metabolic index unit 542, stress marker index unit 550, and physical fitness/integrity unit 555 may be adapted to transmit their respective index values to an SSI value determination unit 530. The SSI value determination unit 530 may use a neurologic index value, an autonomic index value, an endocrine index value, a metabolic index value, a stress marker index value, a physical fitness/integrity index determined by the seizure data, and/or other body data to determine a seizure severity index value (SSI value), as described above with respect to Figure 2. The SSI value may be transmitted/provided to the extreme epileptic event/state detection, quantification and risk determination unit 290, the warning unit 289, the seizure (extreme seizure) ranking unit 247, a memory 217, a database 250/255, a remote device 292, and/or other components of the MD 200. It is noted that in some embodiments the SSI value may be sent directly to the warning unit 289 and/or the seizure (extreme seizure) ranking unit 247 without being sent to the extreme epileptic event/state detection, quantification and risk determination unit 290.

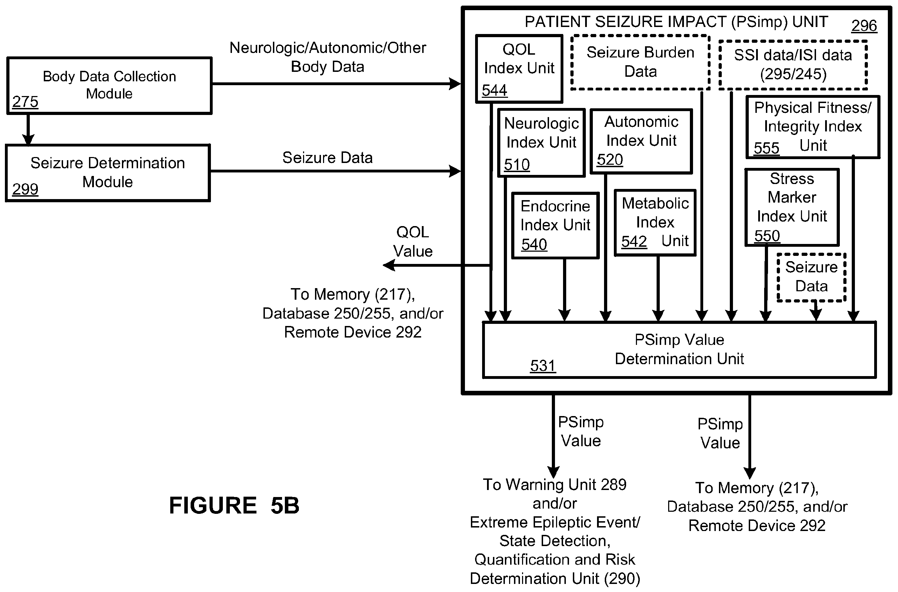

Turning now to Figure 5B, a block diagram depiction of a patient impact unit 296 is provided, in accordance with one illustrative embodiment of the present invention which is more fully described in U.S. patent applications 13/040,996, 13/091,033, 13/333,235, which are incorporated by reference herein in their entirety. In one embodiment, the PSimp unit 296 may be adapted to include a seizure severity index (SSI) unit 295 or to use the data from a separate SSI unit 295. The PSimp unit 296 may, in one embodiment, comprise a QOL index unit 544.

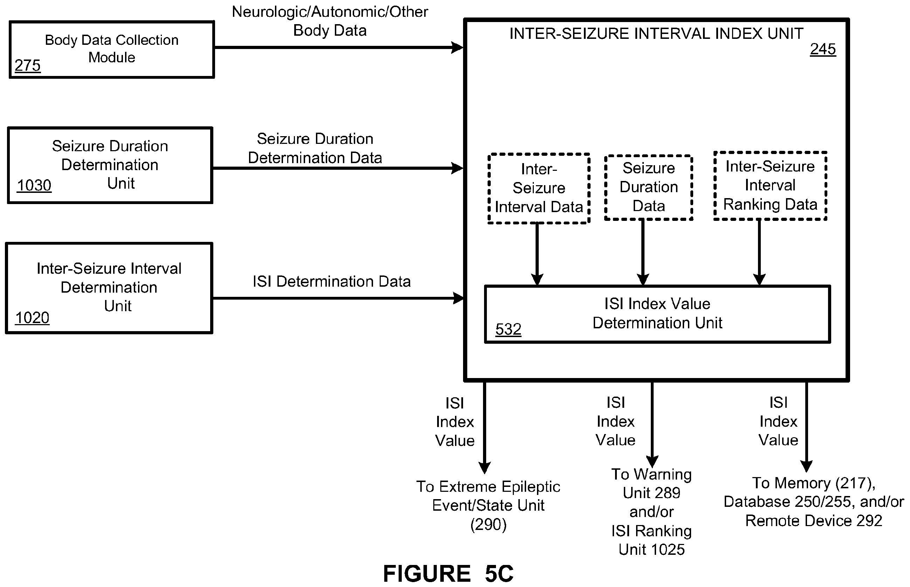

Turning now to Figure 5C, a block diagram depiction of an inter-seizure interval index unit 245 (ISI index unit) is provided, in accordance with one illustrative embodiment of the present invention. In one embodiment, the ISI index value determination unit 532 may use a neurologic index value, an autonomic index value, an endocrine index value, a stress marker index value, seizure data and/or other body data to determine an inter-seizure interval index value (ISI index value), as described above with respect to Figure 2. In one embodiment, the ISI index value may be indicative of extreme seizure events/states if the time interval between two or more seizures is below (by one or more standard deviations) the mean of a normalized distribution of ISIs or at or below the 20^{th} percentile of distribution values. The ISI index value may be transmitted/provided to the extreme epileptic event/state detection, quantification and risk determination unit 290, the warning unit 289, the inter-seizure interval ranking unit 1025, a memory (217), a database 250/255, a remote device 292, and/or other components of the MD 200. It is noted that in some embodiments the ISI value may be sent directly to the warning unit 289 and/or the seizure (extreme seizure) ranking unit 247 without being sent to the extreme epileptic event/state detection, quantification and risk determination unit 290.

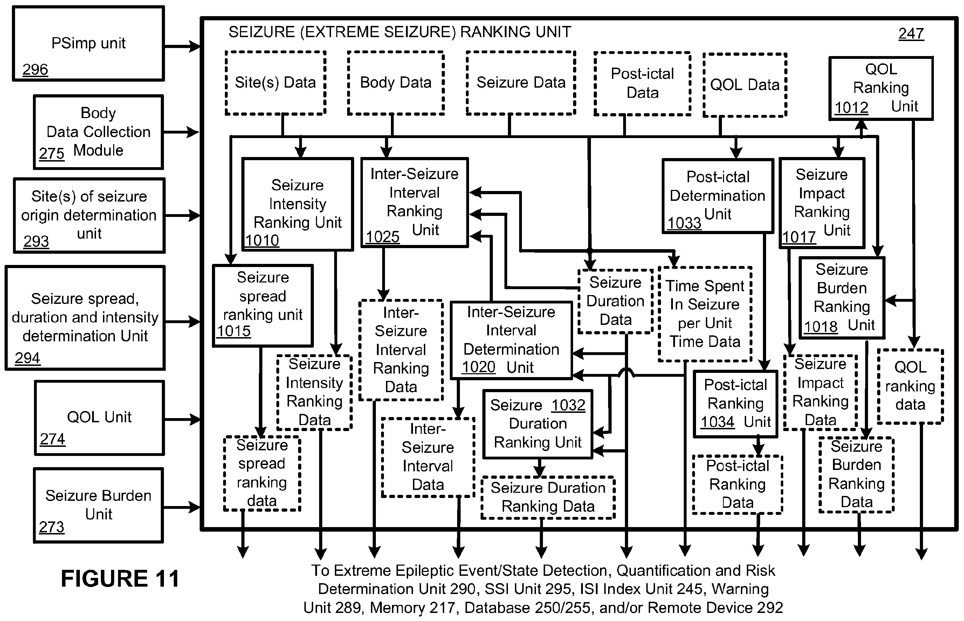

In one embodiment, the ISI index unit 245 may be adapted to determine an inter-seizure interval (ISI). The ISI index unit 245 may use the time of onset and/or termination of at least two consecutive or non-consecutive seizures from a seizure spread, duration and intensity determination unit 294 to calculate the time elapsed between the seizures. In one embodiment, the ISI index unit 245 may receive data from duration determination unit 1030 (see Figure 11 below) and inter-seizure interval data from an inter-seizure interval determination unit 1020 (see Figure 11 below), body data from the body data collection module 275 and/or other seizure data (not shown) in determining the ISI index. In one embodiment, body data collection module 275 may send body data to the ISI index unit 245. Such body data may include, but is not limited to, neurologic and/or autonomic body data, endocrine data, stress marker data, physical activity data, and/or the like. Likewise, the seizure determination module 299 may send seizure data to the ISI index unit 245.

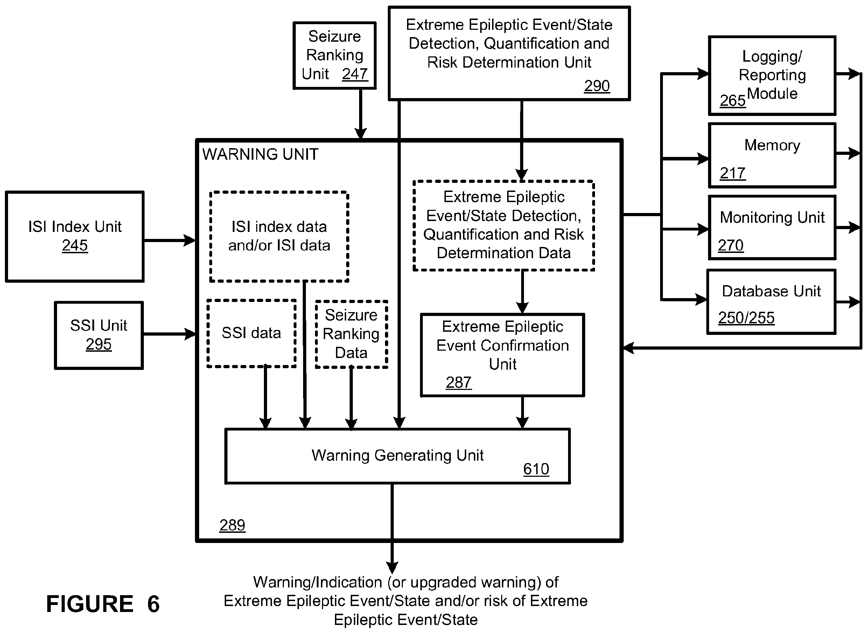

Turning now to Figure 6, a block diagram depiction of a warning unit 289 is provided, in accordance with one illustrative embodiment of the present invention. In one embodiment, the warning unit 289 may be adapted to provide a warning of a seizure, seizure events and/or extreme seizure events. In various embodiments, extreme seizure events may include a present or past state of status epilepticus, an increased risk of status epilepticus, a risk of SUDEP associated with a seizure, an increased risk of SUDEP associated with a seizure, the occurrence of injury or of an increased risk of injury and/or the like. The warning unit 289 may provide a warning to a patient, a physician, a caregiver, the logging/reporting module 265, the monitoring unit 270, the remote device 292, the memory 217, the database 250/255, and/or the like.

The warning unit 289 may include a warning generating unit 610, in accordance with one embodiment. The warning unit 289 may be adapted to receive SSI data from the SSI Unit 295, ISI data from the ISI Index Unit 245, PISI data from the (post-ictal determination unit 1033), physical fitness/physical integrity data from the physical fitness/integrity index unit 555, PSimp data from the PSimp unit 296, extreme epileptic event/state data from extreme epileptic event/state detection, quantification and risk determination unit 290, and/or extreme seizure event confirmation data from extreme epileptic event confirmation unit 287. In various embodiments, the warning unit 289 may be adapted to receive other signals and/or data in addition to, or alternatively to, the aforementioned data, as shown in Figure 6. In one embodiment, the warning generating unit 610 may take any data received by the warning unit 289 as an input to generate a warning. The warning may be a general warning related to a seizure or extreme seizure event or state such as status epilepticus, an upgrade in warning of an existing extreme seizure state/event, or related to an injury associated with a seizure. In one embodiment, the warning unit 289 may include an extreme epileptic event confirmation unit 287. The extreme epileptic event confirmation unit 287 may take data from the extreme epileptic event/state detection, quantification and risk determination unit 290 and confirm or verify that an extreme epileptic event/state is likely, is about to occur, is occurring or has occurred. If the extreme event/state is confirmed, the confirmation may be sent to the warning generating unit 610. If the extreme event/state is not confirmed, the warning may be blocked or disabled, and data associated with the blocked/disabled warning may be removed.

Warnings may include sounds or lights, automated emails, text messages, telephone calls, or video messages sent from the MD 200, either directly or via a monitoring unit 270, to the police, an EMT unit, the patient's physician/caregiver's cellular telephone, PDA, computer 150, television, etc. Such warning(s) may allow the patient and/or caregivers to take measures protective of the patient's well-being and those of others, *e.g*., pulling out of traffic and turning off a car, when the patient is driving; stopping the use of machinery, contacting another adult if the patient is providing childcare, removing the patient from a swimming pool or bathtub, lying down or sitting if the patient is standing, etc. The warning may, when appropriate, automatically disable operation of a vehicle or of power equipment or inflate a life saver (*e.g.,* for a patient who is swimming) or bags placed on the chest or back of a patient to minimize risk of injury in case of falls.

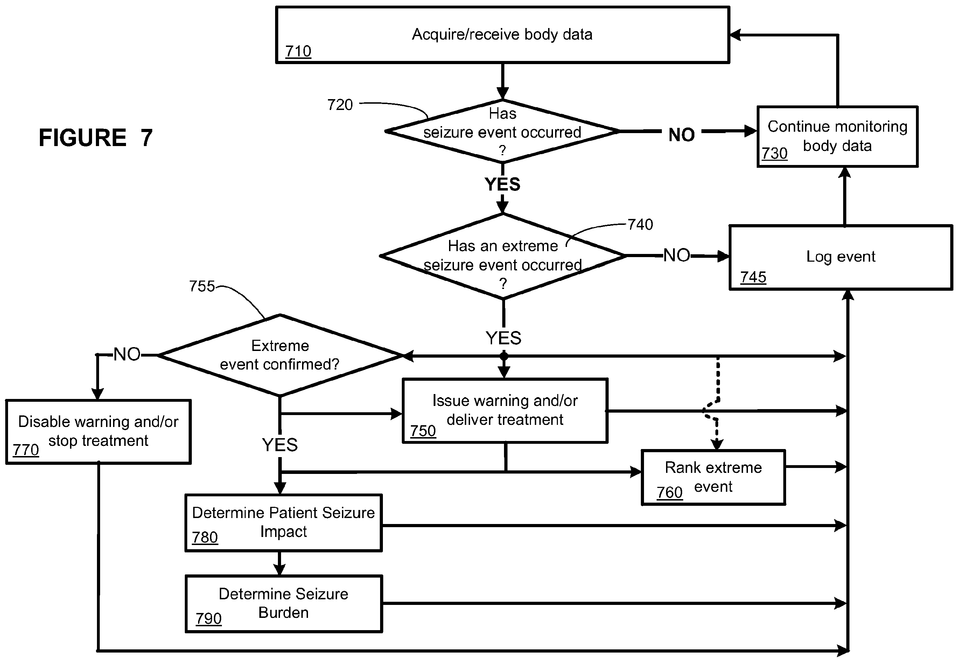

Turning now to Figure 7, a flowchart depiction of a method for taking action (*e.g.,* warning, providing treatment/therapy, logging, etc.) in response to the occurrence of a seizure event and/or an extreme seizure event is provided, in accordance with one illustrative embodiment of the present invention. The MD acquires and/or receives body data at 710, typically from the body data collection unit 275 which buffers, amplifies/conditions and performs A/D conversion of the body data. Using data from unit 275, the seizure determination module 299 in the MD 200 determines, through operations including but not limited to calculations based on at least one index, if a seizure event has occurred, is likely to occur or is occurring (720). If the MD 200 determines that no seizure or seizure event has occurred, the MD 200 will continue to monitor for body data (730; return the flow to 710).

If the medical device determines (at 720) that a seizure event has occurred or is occurring or is likely to occur, based on at least one of an autonomic index, a neurologic index, a stress marker index, a metabolic index and/or an endocrine index (*e.g*., using an SSI unit 295, an ISI index unit 245, a PSimp unit 296 and/or another unit/module in the MD 200), at 740, it may be to determine, *e.g*., using unit 290, if the event is extreme. Determination that the event is extreme, may trigger responsive actions (750) including but not limited to delivering a therapy or therapies, (unit 220), warning(s) (unit 289) and requesting confirmation via unit 287 that the event is extreme 755), and if it is deemed extreme, it may be then ranked 760), typically using unit 247. If the event is not confirmed as extreme, all responsive actions may be terminated 770). At any given action in the flowchart, information yielded by such an action, and the decisions made based on this information, may be sent to logging/reporting unit 265 at 745) and/or to memory 217. 750, 760 and 770 may begin at the same time and end at the same time (or at different times) according to various embodiments. Further, it is contemplated that events may be logged after a determination and/or confirmation of an extreme event (at 740/755) has occurred.

The number of actions and the order in which they are adopted may vary according to one of several possible embodiment contemplated herein. For example, confirmation that the event is extreme may be unnecessary if all indices (autonomic, neurologic, endocrine, metabolic, tissue stress marker, physical integrity, etc.) are determined simultaneously. However, if all indices are not measured, those untested may be determined in any number and/or temporal sequence for the purpose of confirmation. The medical device 200 may determine an SSI value using at least one of an autonomic index, a neurologic index, a stress marker index, a metabolic index and/or an endocrine index; the medical device 200 may determine an inter-seizure interval index (ISI index) value using at least one of an autonomic index, a neurologic index, a stress marker index, a metabolic index and/or an endocrine index; the medical device 200 may determine a physical fitness/integrity index value using at least one of an autonomic index, a neurologic index, a stress marker index, a metabolic index and/or an endocrine index. Typically, the SSI value is determined by SSI unit 295 (which may comprise an SSI determination unit (530)). Typically, the ISI index value is determined by ISI index unit 245. Typically, the physical fitness/integrity index value is determined by physical fitness/integrity index unit 355. In one or more embodiments, additional data may also be used to determine the ISI index value and/or physical fitness/integrity index value. Depending upon the embodiment, one or more of the SSI value, the ISI index value and/or the physical fitness/integrity index value may be determined. For example, in one embodiment, only the SSI value may be calculated while the ISI index value is not calculated. In another embodiment, only the ISI index value may be calculated while the SSI value is not calculated. In one or more embodiments, additional data may also be used to determine the SSI values, ISI values and/or physical fitness/integrity index values.

The confirmation of an extreme epileptic event/state parameter(s) may be performed by an extreme epileptic event/state confirmation unit 287 (at 755). The confirmation of an extreme epileptic event/state may be based upon one or more SSI values and/or one or more ISI index values as described above, and/or be based upon other data/indices as described above. From 755, the flow may proceed to determining a patient seizure impact (PSimp) value 780), and in some embodiments, the flow may then proceed to determining a seizure burden value 790). The medical device 200 may determine a PSimp value using at least one of an autonomic index, a neurologic index, a stress marker index, a metabolic index and/or an endocrine index, a quality of life index or a physical fitness/integrity index and or the like 780). The medical device 200 may determine a seizure burden value using at least one of an autonomic index, a neurologic index, a stress marker index, a metabolic index and/or an endocrine index and/or the like 790). Other procedures/modules may be used to determine a seizure burden and/or patient seizure impact. For example, an Acquire/Determine Physical Fitness Index/Body Integrity Unit (not shown) may use these data to determine seizure burden and/or patient seizure impact. The MD 200 may also take responsive action for extreme epileptic event/state (at 750) that may include, but is not limited to, drug/chemical therapy, electric stimulation, cooling, supportive care, oxygen administration, warning, logging/reporting, and/or the like. It is also contemplated that a patient's physical activity may be stored in a memory 217 or logged/reported in one or more of the database 250/255 and the remote device 292.

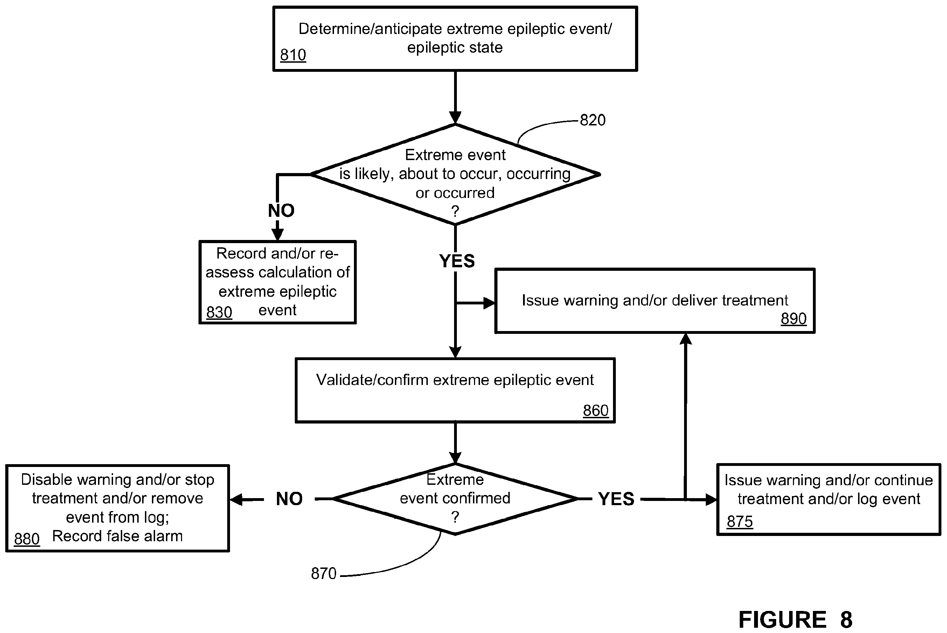

Turning now to Figure 8, details of the actions in Fig 7 that are implemented when an extreme event is likely to occur, about to occur, is occurring or has occurred are provided, is depicted in accordance with one or more illustrative embodiments. The MD 200 may issue a warning or take action 890) based upon detection of an extreme epileptic event/state in a patient (s810/820). It should be noted that in some embodiments an extreme epileptic event is the occurrence of an actual extreme epileptic seizure or condition, while in other embodiments the extreme epileptic event is an elevated risk of an extreme epileptic seizure or condition. If the MD 200 determines a patient is not in an extreme epileptic event/state 820), the MD 200 may proceed to record the current condition of the patient and reassess calculation(s) of extreme epileptic event/state data (830). If the MD 200 determines a patient is in an extreme epileptic event/state 820), the MD 200 may in some embodiments perform an extreme epileptic event confirmation 860). If the MD 200 determines a patient is in an extreme epileptic event/state 820), the MD 200 may, in one embodiment, issue a warning and/or deliver treatment to the patient 890).

In one embodiment, the MD 200 may delay warning the patient until the extreme seizure event confirmation is confirmed 860). It is contemplated that 860 and 890 may occur at the same time or at different times in various embodiments described herein. If the MD 200 determines an extreme seizure event confirmation has not occurred (870), the MD 200 records and/or logs a tentative and/or potential false positive event, *e.g.,* by recording the original detection as erroneous or unconfirmed 880), if this determination has not been based on all indices disclosed in this invention. In the case that all disclosed indices have not been used for said determination, one or more indices not used in issuing a "false positive" decision may be measured and depending on the results of the more comprehensive analysis, the decision ("false positive") may be confirmed or rejected. If a false positive event is issued, the medical device 200 may also disable (at least transiently) an issued warning and/or stop treatment and/or remove an event from the log 880). If a false positive event is confirmed, this outcome may be recorded and/or logged in a logging/reporting module 265 880). If the false positive event is rejected, an extreme event detection may be re-issued and all responsive actions will be re-instituted according to the embodiments of this invention (*e.g*., 870, 890).

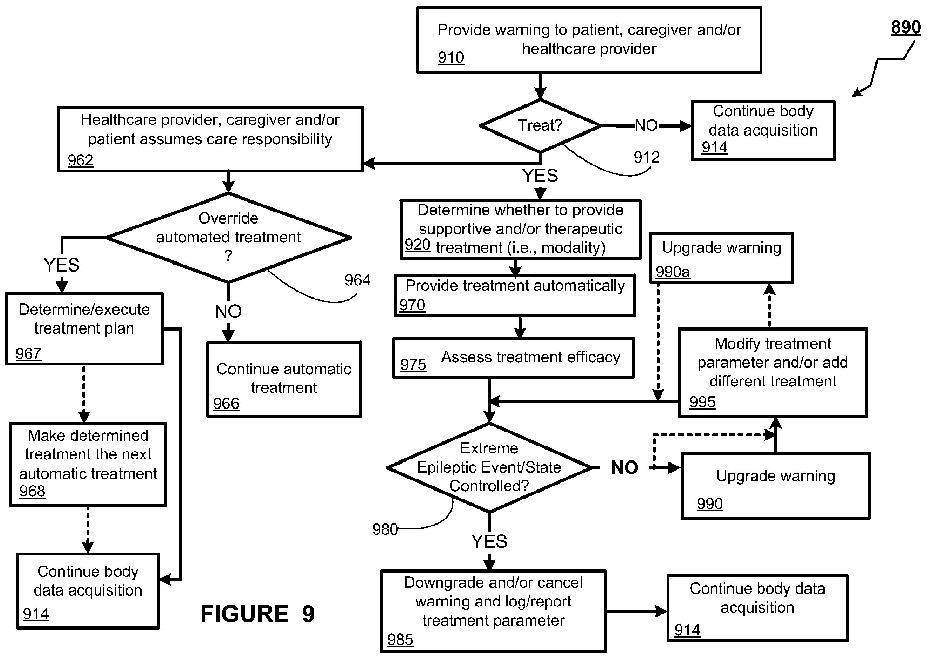

Turning now to Figure 9, a flowchart depiction of a method for warning and/or providing a treatment to a patient in response to an extreme seizure event is provided, in accordance with one illustrative embodiment. The MD 200 may provide a warning of a state of an extreme epileptic event/state via the warning unit 289 in one or more embodiments 910). The warning may be to a patient, a physician, a caregiver, emergency response personnel, a logging/reporting module 265, a monitoring unit 270, a remote device 292, an external entity 265 and/or the like. The warning may indicate an extreme epileptic event/state (*e.g*., a severe seizure, status epilepticus, or an injury resulting from an otherwise non-extreme seizure). At 912, the decision is made whether or not to treat the patient based upon the warning from 910. If it is determined that no treatment will be performed, the flow proceeds to 914 where patient monitoring and body data acquisition continues. If it is determined that treatment will be administered, the flow may proceed to one or both of 920 and/or 962.

A determination may be made as to which treatment modality(ies) are to be provided to a patient 920). Modalities include, but are not limited to, electrical currents, chemical/drug therapies and/or supportive treatments such as cooling, fluids, pressor agents and/or oxygen. In one embodiment, the MD 200 may automatically implement a predetermined treatment to reduce the risk of an extreme epileptic event/state and/or to reduce the effects of a state of an extreme epileptic event/state in the patient 970) using one or more treatment modalities. In reference to supportive care, seizures are powerful biological stressors and inductors of stress markers and may deplete the body of certain anti-oxidants such as glutathione peroxidase. The concentration of certain compounds that protect from biological stress (e.g., dehydroepiandrosterone or its sulfate conjugate, glutathione peroxidase and/or the like) or the body's total antioxidant capacity may be measured to determine if it is adequate, and if not, to increase it using available antioxidants to preserve the integrity of organs/functions so as to stall disease progression. Stress marker index indices and antioxidants may be measured in brain (invasively and/or non-invasively), CSF, plasma, serum, erythrocytes, urine, and saliva (*e.g*., alpha amylase).

Upon delivery of automatic treatment(s), an assessment of the efficacy of the treatment may be performed 975) in some embodiments. Based upon the assessment of the efficacy of treatment, a determination is made whether the state of the extreme epileptic event/state is at least substantially controlled 980). If a determination is made that the extreme epileptic event/state is not substantially controlled 980), the MD 200 may upgrade the warning to a more severe level 990) and the treatment being delivered may be modified, and/or an additional treatment may be provided 995). In an alternative embodiment, upgrading the warning may be initially omitted and the treatment may be modified first, as indicated by the dotted line connecting blocks 980 and 995 and 990a. The MD 200 may then continue to determine whether the state of the status epilepticus is substantially controlled 980).

Upon a determination that the extreme event/state is substantially controlled 980), the warning may be downgraded and/or canceled 985). Further, the treatment parameter(s) used for the administration of the automatic treatment may be reported/logged 985). The MD 200 may then continue to perform body data acquisition 914).

In one embodiment, upon providing a warning to the patient, caregiver, and/or to a healthcare provider 910), the MD 200 may provide for the healthcare provider, the caregiver, and/or the patient to assume care/treatment responsibilities 962). Based upon one or more inputs received by the MD 200, a determination may be made as to whether to override the automated treatment 964). If it is determined that the automated treatment is not to be overridden, then the state of the automatic treatment is maintained 966).

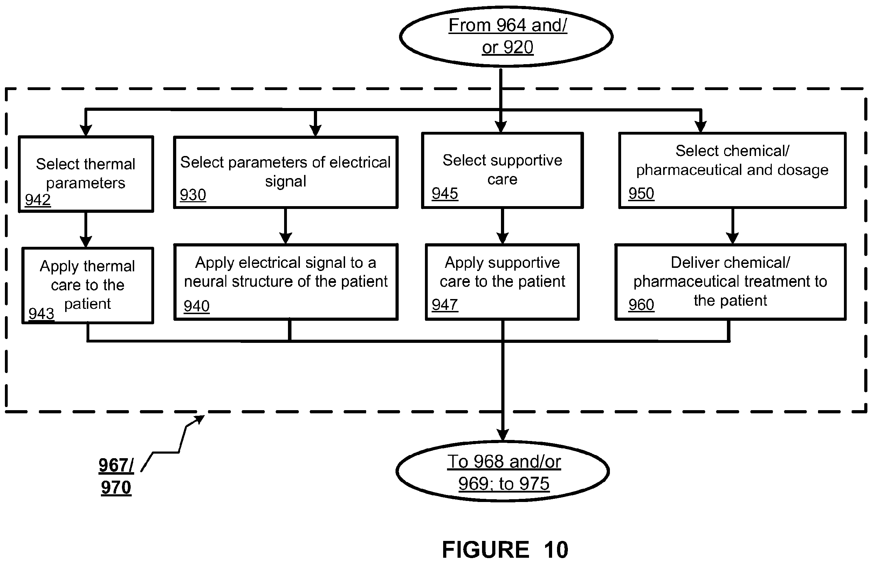

If a determination is made that the automated treatment is to be overridden, a non-automated treatment plan is determined and executed 967). A more detailed description of the determining and executing a non-automated treatment plan is provided in Figure 10 and accompanying description below. In one embodiment, upon determining and executing a non-automated treatment plan, this treatment plan may be set as the next automatic treatment that is executed by the MD 200 968). The MD 200 then continues to perform body data acquisition 914). In an alternative embodiment, setting the non-automatic treatment plan as the default automatic treatment plan may be omitted, as indicated by the dotted lines between blocks 967, 968 and 969.

Turning now to Figure 10, a stylized depiction of determining an automatic or a non-automatic treatment plan of 967/970 of Figure 9, in accordance with one illustrative embodiment, is provided. The MD 200 may select a chemical/drug the dosage and rate of delivery 950) and deliver the drug/chemical to the patient 960). In addition to, or alternatively to, the chemical treatment, the MD 200 may select parameters of an electrical signal to treat the patient 930) and apply the specified electrical signal to a neural structure (*e.g*., a branch of the vagus nerve) of the patient 940). Further, in addition, or alternatively, the MD 200 may select one or more thermal parameters to specifically treat the extreme event/state with a thermal therapy (heating and/or cooling) 942) and apply it 943). In alternative embodiments, additional and/or other treatment modalities may be implemented, such as endosomatic therapies (*e.g*., biofeedback, sensory, and/or cognitive stimulation). The flow may then proceed to 968/969 and/or 975. Further, in addition, or alternatively, the MD 200 may select one or more supportive care actions to treat the patient 945) and apply the specified supportive care 947).

Turning now to Figure 11, a block diagram depiction of a seizure (or extreme seizure) ranking unit 247 is provided, in accordance with one illustrative embodiment. Rankings reduce in-depth measures to a sequence of ordinal numbers, thus facilitating the evaluation of complex information according to certain criteria. Rankings, in embodiments described herein, may be done according to one or more of a so-called standard completion, modified, ordinal, dense or fractional ranking. Those skilled in the art having the benefit of this disclosure would appreciate the approach to ranking described herein, is applicable to the ranking of any measure or index in this invention.

In one embodiment, the seizure (extreme seizure) ranking unit 247 may rank events classified as extreme using data from the extreme epileptic event/state detection, quantification and risk determination unit 290 and may provide the rankings to the memory (217), database 250/255, and/or remote device 292. In various embodiments, the seizure (extreme seizure) ranking unit, may receive data directly from, for example, one or more units shown in Figure 2 (described above), about seizure intensity data, seizure intensity rankings, inter-seizure interval data, inter-seizure interval ranking data, seizure duration data, time spent in seizure data, and/or the like. In one or more embodiments, the output of the seizure (extreme seizure) ranking unit 247 may be an indication of a seizure ranking as compared to historical seizure rankings and/or one or more reference values. This may be accomplished by first determining if an SSI and/or an ISI value are near, at, or outside the boundaries between extreme and non-extreme values. If an event fulfills the criteria to be classified as extreme, it is ranked relative to other extreme events, and the warning, treatment, etc., are upgraded based on said ranking (*e.g*., said event ranks above the 60^{th} percentile for SSI and below the 40^{th} percentile for ISI). For a normalized distribution the upgrade may take place if the extreme event is for example, one SD to the right of the mean or median for SSI and one SD to the left of the mean or median for ISI. It is contemplated that other percentile ranking values may be used.

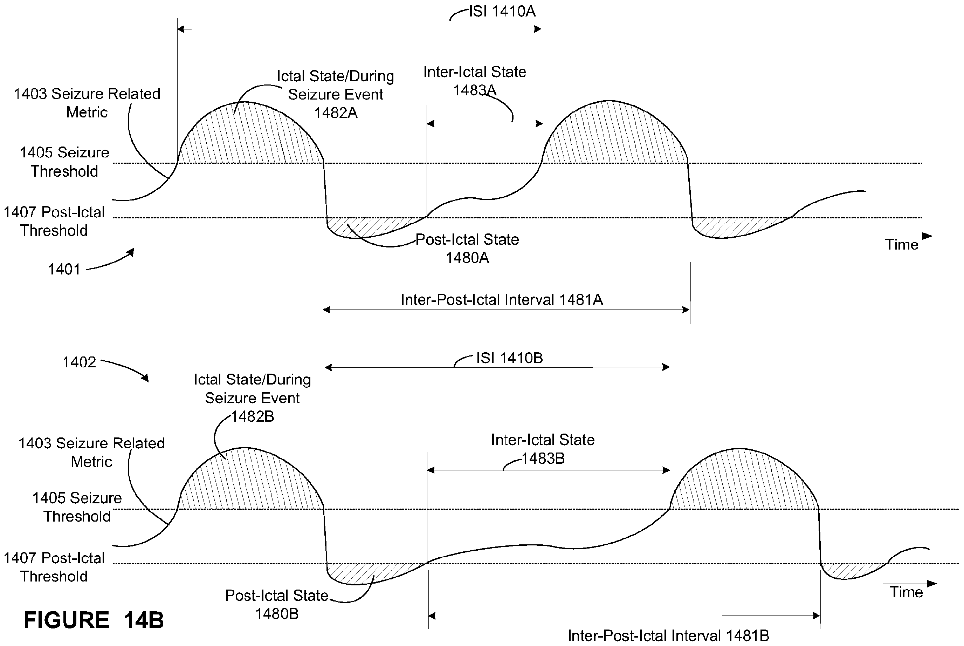

The seizure (extreme seizure) ranking unit 247 may include a seizure intensity ranking unit 1010, in accordance with one or more embodiments. The seizure intensity ranking unit 1010 may be adapted to receive body data from a variety of units as shown in Figure 2 (described above). It should be noted that these determinations of intensity recited herein are not exclusive, and the determination of seizure intensity data using other body data is also contemplated, as would be evident to one of skill in the art having the benefit of this disclosure. Seizure intensity data may be determined from site(s) of seizure origin data and/or at each site (brain and/or body) to where the seizure spreads or is manifested, whether directly or indirectly. In various embodiments, the seizure intensity ranking unit 1010 may determine seizure intensity data based upon data measurements over a period of time or body data as a function of time (as discussed in further detail below with respect to Figures 14-15, or based only on one of intensity, duration or extent of spread. That is, intensity, duration and extent of spread may be ranked separately from SSI by the seizure (extreme seizure) ranking unit 247. This approach allows classification of any of these three metrics as extreme, even if a composite measure incorporating all three (*e.g.,* SSI = (percentile intensity + percentile duration + percentile spread) / 3) may not be extreme. This may allow for better anticipation and for prevention of extreme epileptic events by allowing seizures to be classified as extreme based on one of the three metrics alone, even though the SSI may not be extreme based on two or more of them. Similarly, it may be advantageous to issue a certain kind of warning (different than the warning for an extreme SSI) and deliver a therapy in this instance. As such, it may be noted that there are: 1) extreme intensity and/or duration and/or spread; and/or 2) extreme events/states when the SSI is extreme.

The seizure (extreme seizure) ranking unit 247 may include a seizure duration ranking unit 1032, in accordance with one or more embodiments. The seizure duration ranking unit 1032 may be adapted to determine seizure ranking data which may include the ranking of a patient's seizure event with respect to a reference value and/or compared with past seizure/extreme seizure events/states.

The seizure (extreme seizure) ranking unit 247 may include a seizure spread ranking unit 1015, in accordance with one or more embodiments. The seizure spread ranking unit 1015 may be adapted to determine a seizure spread ranking, *i.e.,* a relative determination of a seizure spread relationship to past values.

The seizure (extreme seizure) ranking unit 247 may include an inter-seizure interval ranking unit 1025, in accordance with one or more embodiments. The inter-seizure interval ranking unit 1025 may be adapted to receive ISI data from the ISI determination unit 1020 (described below) and/or other data from various components of the MD 200. The inter-seizure interval ranking unit 1025 may be adapted to determine an inter-seizure interval ranking, *i.e.,* a relative determination of a seizure interval relationship to past interval values. In one embodiment, for example, the inter-seizure interval ranking may be based upon a normal/normalized distribution of seizure interval values. The beginnings and ends of seizure events may be determined based upon body data, site(s) data and/or spread data values relative to dynamic or pre-determined thresholds, responsiveness of a patient, and/or the like.

The seizure (extreme seizure) ranking unit 247 may include an inter-seizure interval ISI) determination unit 1020, in accordance with one or more embodiments. The inter-seizure interval determination unit 1020 may be adapted to receive body data from various components of the MD 200. The inter-seizure interval determination unit 1020 may be adapted to determine an inter-seizure interval (ISI), *i.e.,* a time period between seizure events. In various embodiments, the inter-seizure interval may be measured from the end of one seizure event to the beginning of the next seizure event and/or from the onset of a first seizure to the onset of a second seizure. The beginnings and ends of seizure events may be determined based upon body data, site(s) data and/or spread data values relative to adjustable or pre-determined thresholds, responsiveness/awareness of a patient, and/or the like. In one embodiment, the time spent by a patient in a seizure event state (or the time spent in a seizure) may be quantified as one or more of a seizure duration plus ISIs or portions thereof. In one or more embodiments, an epileptic event may be classified as extreme based upon on the ISI, or based upon the ISI in addition to other values described herein.

In various embodiments, seizure duration data, seizure ranking data and/or the time spent in seizure data may be logged/reported in logging/reporting module 265 and/or transmitted to the extreme epileptic event/state detection, quantification and risk determination unit 290, the SSI unit 295, memory 217 and/or database 250/255 as described above with respect to Figure 2.

The seizure (extreme seizure) ranking unit 247 may include a post-ictal ranking unit 1034, in accordance with one or more embodiments. The post-ictal ranking unit 1034 may be adapted to determine post-ictal ranking data which may include the ranking of a patient's post-ictal state with respect to a reference value and/or compared with past post-ictal states.

The seizure (extreme seizure) ranking unit 247 may include a post-ictal determination unit 1033. In one embodiment, the post-ictal state may be described as the state in which the values of any of the indices disclosed herein are different from those observed in the inter-ictal and ictal states. In accordance with one embodiment, the post-ictal determination unit 1033 may determine the duration of a post-ictal state of a patient. The post-ictal index determination 1033 may also determine a severity of the post-ictal state based upon the magnitude and/or duration and or spread of the post-ictal state, as well as the rate of change into and out of a post-ictal state, in manners similar to those used in the described embodiments herein for determining SSI.

The seizure (extreme seizure) ranking unit 247 may include a seizure impact (PSimp) ranking unit 1017, in accordance with one or more embodiments. The seizure impact ranking unit 1017 may be adapted to determine a seizure impact ranking, *i.e.,* a relative determination of a seizure impact relationship to past values..

The seizure (extreme seizure) ranking unit 247 may include a QOL ranking unit 1012, in accordance with one or more embodiments. The seizure (extreme seizure) ranking unit 247 may include a seizure burden ranking unit 1018, in accordance with one or more embodiments. The seizure burden ranking unit 1018 may be adapted to determine a seizure burden ranking, *i.e.,* a relative determination of a seizure burden relationship to historical interval values, normative values and/or expected values.

In one or more embodiments, the seizure duration ranking unit 1032, seizure spread ranking unit 1015, the seizure intensity ranking unit 1010, the seizure spread, duration and intensity determination unit 294, inter-seizure interval ranking unit 1025, the inter-seizure interval determination unit 1020, QOL ranking unit 1012, QOL unit 274, post-ictal ranking unit 1034, post-ictal determination unit 1033, PSimp ranking unit 1017, PSimp unit 296, seizure burden ranking unit 1018, seizure burden unit 273 and/or site(s) of seizure origin determination unit 293 may transmit information to and from each other for the purposes of determining their respective data outputs. Further description of the various ranking units above may be found in U.S. patent applications 13/040,996, 13/091,033, 13/333,235, which are incorporated by reference herein in their entirety.

Seizure duration, intensity, extent of spread, ISI and all of their mathematical transformations, seizure burden and PSimp may be analyzed and interpreted in the context of for ultradian, circadian, or infradian rhythms (if present) and if trends are observed, said trends may be used to improve estimation of risks of having an extreme event. The efficacy of therapies may be also assessed in the context of these rhythms and optimized as needed, using these observations.

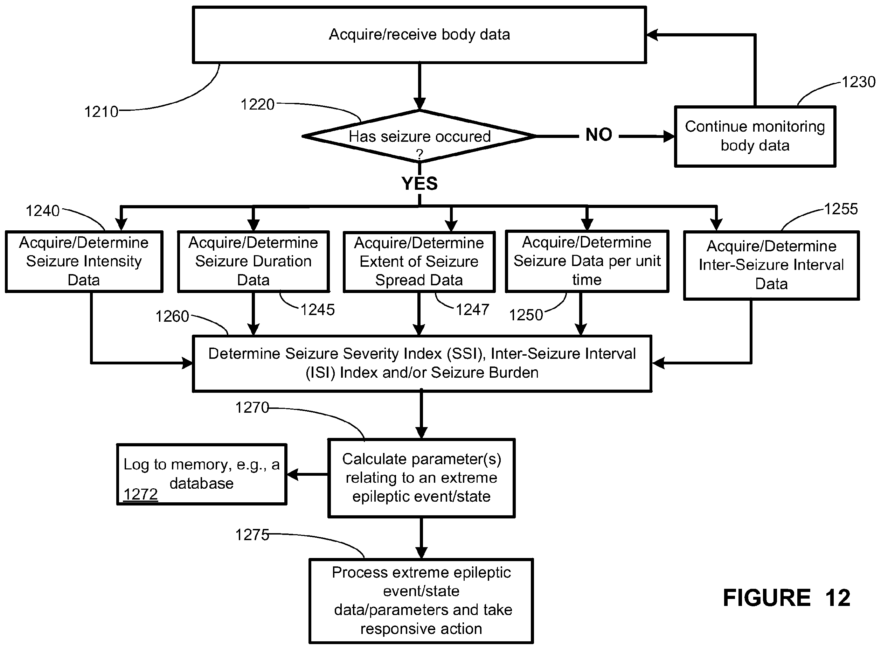

Turning now to Figure 12, a flowchart depiction of a method for taking action (warning, treating and/or logging) in response to determining a seizure event (extreme seizure event/state) is provided, in accordance with one illustrative embodiment of the present invention. The medical device 200 acquires and/or receives body data at 1210). Typically, the body data collection unit 275 receives the body data. The body data may be indicative of whether or not a seizure or extreme seizure event has occurred or is occurring. After performing buffering, amplification/conditioning and A/D conversion of the body data, the medical device 200 determines if a seizure is about to occur, is occurring or has occurred 1220). Typically the seizure determination module 299 makes this determination based upon one or more calculations. If the medical device 200 determines that no seizure or seizure event has occurred, the medical device 200 will continue to monitor for body data 1230 and return the flow to 1210).

If the medical device determines (at 1220) that a seizure is about to occur, is occurring or has occurred, the method proceeds to acquire seizure intensity data 1240), seizure duration data 1245), extent of seizure spread data 1247), time spent in seizure data 1250) and/or inter-seizure interval data 1250). In one embodiment, indices are acquired and/or determined using an SSI unit 295 (typically comprising an autonomic index unit 520, a neurologic index unit 510, an endocrine index 540, a metabolic index 542 and/or a tissue stress marker index unit 550). Actions 1240, 1245, 1247, 1250 and/or 1255 may begin at the same time and end at the same time (or at different times and in different combinations) according to different embodiments contemplated herein. In other words, actions 1240, 1245, 1247, 1250 and/or 1255 may begin and be completed substantially in parallel (*i.e.,* at approximately the same time or at the same time) or independently of each other. The medical device determines an SSI value, an ISI index value and/or a seizure burden value using the seizure intensity data, seizure duration data, time spent in seizure data and/or inter-seizure interval data 1260). Typically, the SSI value is determined by SSI unit 295 (which may comprise an SSI determination unit (530), as described above with regard to Figure 11). In one or more embodiments, additional data may also be used to determine the SSI value.

Still referring to Figure 12, once an SSI value is determined 1260), the flow may proceed to 1270. The medical device 200 calculates one or more parameters related to an extreme epileptic state/event 1270). The calculation of extreme epileptic state/event parameter(s) may be performed by an extreme epileptic event/state detection, quantification and risk determination unit 290. The calculation of extreme epileptic state/event parameter(s) may include a calculation using one or more SSI values. Upon calculating extreme epileptic state/event parameter(s), the MD 200 may log the parameters into memory, *e.g*., in a database 1272). The extreme epileptic state/event parameter(s) may be stored in an external memory (*e.g*., the database unit 250 and/or the local database unit 255), and/or in memory that is internal to the MD 200 (*e.g*., memory 217). The status epilepticus parameters may also be sent to an external device 265. From 1270, the flow may proceed to the processing of extreme epileptic state/event data 1275). The medical device 200 may also take responsive action (at 1275) such as a warning performed by warning unit 289 which may comprise a warning generation unit 610. In one or more embodiments, the responsive action may be taken according to (or similar to) the flowchart depicted in Figures 7-9 and 12.

Turning now to Figure 13, a seizure energy probability density function (PDF) is depicted, according to one embodiment.

Turning now to Figure 14A, one example of a probability density function of inter-seizure intervals (ISI) is illustrated

Turning now to Figure 14B, a graphical depiction of the temporal boundaries/metrics of: three states of interest: a) inter-ictal (described as the period of time during which the value of an index is different from ictal and post-ictal values); b) ictal (described as the period of time during which the value of an index is different from the inter-ictal and post-ictal values) and c) post-ictal (described as the period of time during which the value of an index is different from inter-ictal and ictal values) is provided, in accordance with one illustrative embodiment. Figure 14B shows a seizure related metric 1403 in a two-threshold (1405/1407) graphical representation of two illustrative seizure events: 1401 and 1402. The depicted seizure events 1401 and 1402 have ictal states 1482A and 1482B respectively and post-ictal states 1480A and 1480B respectively. In between seizure events 1401 and 1402 lie inter-ictal states 1483A and 1483B respectively. As illustrated, when the seizure related metric 1403 reaches/crosses the seizure threshold 1405 in an upward direction, the patient is said to be in an ictal state (1482A/1482B). When the seizure related metric 1403 reaches/crosses the post-ictal threshold 1407 in a downward direction, the patient is said to be in a post-ictal state (1480A/1480B) and when it crosses/reaches in an upward direction post-ictal threshold 1407 (without reaching/crossing ictal threshold 1405), the patient is said to be in an inter-ictal state.

Figure 14B also depicts an inter-seizure interval (ISI) 1410A and 1410B, as well as inter-post-ictal intervals 1481A/1481B, for each illustrated seizure event 1401 and 1402 respectively, in accordance with one or more one embodiments. The illustrated ISI 1410A may be described as the time elapsed between the onset of two consecutive (or non-consecutive) seizures or ictal states and the ISI 1410B may be described as the time elapsed between the end of a seizure or ictal state and the onset of consecutive (or non-consecutive) seizures or ictal states, as shown in Figure 14B. An ISI may also be described as the time elapsed between the end of a post-ictal state and the onset of consecutive (or non-consecutive) seizures or ictal states (shown as inter-ictal state 1483A/1483B). Similarly, the illustrated inter-post-ictal intervals 1481A may be described as the time elapsed between the onset of two consecutive (or non-consecutive) post-ictal states and the inter-post-ictal intervals 1481B may be described as the time elapsed between the end of a post-ictal state and the onset of the next post-ictal state. The inter-ictal periods described herein are not exclusive, and other measures may be used as would be realized by one of ordinary skill in the art having the benefit of this disclosure.

As illustrated, the ISI 1410A has a shorter length than the ISI 1410B. In other words, the ictal states 1482A in seizure event 1401 are closer together in time than the ictal states 1482B in seizure event 1402. A shorter ISI may be an indication of a greater overall risk of having an extreme event and also of a higher burden and/or impact; progressive decrease (*e.g.,* a trend) in ISI duration may be indicative of an increased risk for an extreme epileptic event/state. The longer the ISI (usually associated with longer inter-ictal periods) the greater the probability for recovery of body organs/systems to the inter-ictal state (baseline). The temporal length of ISIs may also be indicative of the overall probability of seizure occurrence. That is, in accordance with one or more embodiments, the longer the period between ictal states the lower the probability of a seizure occurring in a patient (*see* Figure 17B below). In contrast, the shorter the ISIs, the higher the probability of a seizure occurring in a patient. The definitions of inter-ictal, ictal and post-ictal states apply to any and/or all autonomic, neurologic, metabolic, endocrine, tissue stress markers and quality of life indices. For example, heart rate, responsiveness/awareness, arterial pH, prolactin, CK, or quality of life during a seizure may be used to determine the onset and termination of the ictal and post-ictal states and the time of return to the inter-ictal state. This approach takes into account all patho-physiologic changes before the onset, during and following the termination to better track their "spatial" (referring to organs/systems spread) and temporal evolution, thus allowing the determination of each state according to each organ/system and each index.

Additionally, an increase above a threshold (*e.g*., seizure threshold 1405) may indicate an increased energy level in certain frequency bands related to a seizure event and/or extreme seizure event in a patient. A decrease below a threshold (*e.g*., post-ictal threshold 1407) may indicate a decreased energy level in certain frequency bands related to a seizure event and/or extreme seizure event in a patient. It is noted that both increases and decreases in energy of various frequency bands related to seizure events and/or extreme seizure events in a patient may provide information related to a body organ from which the energy level/frequency band was recorded.

Longer times spent in post-ictal states (*e.g*., 1480A/1480B) typically may be the result of a more severe seizure, a decline in a patient's overall health or brain health, longer periods of unresponsiveness, an increased risk of injury, status epilepticus and/or SUDEP, and the like. Large magnitude crossings below the downward post-ictal threshold 1407 typically may indicate similar risks to the patient. Additionally, the time spent in post-ictal states (*e.g*., 1480A/1480B) may be analyzed with and compared to the corresponding inter-seizure interval (ISI) and/or as inter-post-ictal intervals (*e.g*., 1481A/1481B) of a seizure event to assess trends of seizure severity and/or intensity.

In one embodiment, the post-ictal state (*e.g*., 1480A/1480B) severity may be used to determine and warn of a risk of an extreme epileptic event/state. In an exemplary embodiment, a downward crossing of a post-ictal threshold 1407 representing the ninetieth percentile of post-ictal severity may indicate an increased risk of an extreme epileptic event/state requiring a warning and/or other treatment/response. It is contemplated that different percentile values may be used to set the downward crossing post-ictal threshold 1407, and that the downward crossing post-ictal threshold 1407 may be adaptable. It is also contemplated that the thresholds 1405/1407 may be based upon a pre-determined or adaptable number of standard deviations of a normalized distribution of post-ictal severity of a patient. For example, if a value meets or exceeds three standard deviations from normal (*i.e.,* the threshold may be seen as three standard deviations from normal), this may indicate that a warning of a risk/occurrence of an extreme epileptic state/event may be needed.

Turning now to Figure 15, a temporal distribution of the probability of seizure occurrence before and after a seizure, in accordance with one embodiment, is depicted.

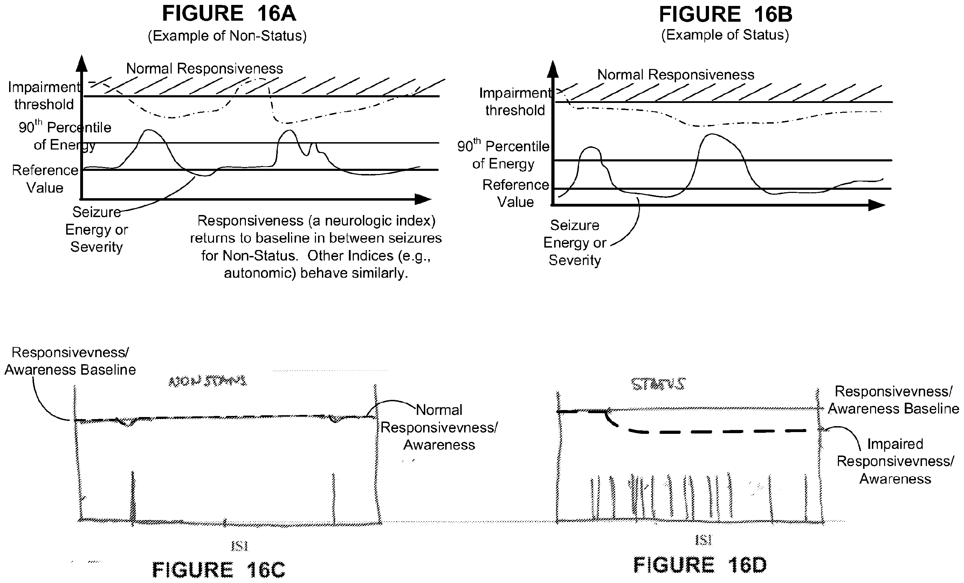

Turning now to Figures 16A and 16B, a relationship between metrics (in this example between responsiveness/awareness (*e.g.,* based on cognitive tests) and seizure severity determined for example using heart rate) is depicted for a non-extreme epileptic state/event (Figure 16A) and an extreme epileptic state/event (Figure 16B), in accordance with one embodiment. As shown in Figure 16A, responsiveness/awareness, returns to its inter-ictal values before the next seizure. It should be noted that a recovery of responsiveness/awareness to baseline or inter-ictal levels in between seizures is associated with a low probability of extreme event/state occurrence. In contrast, Figure 16B depicts a seizure energy increasing to an extreme value (*e.g*., above the 90^{th} percentile of seizure energy values for that patient), indicative of an extreme epileptic event (*e.g*., status epilepticus). Notice that responsiveness/awareness does not recover to inter-ictal levels before the next seizure which further depresses responsiveness.

Turning now to Figures 16C and 16D, a relationship between responsiveness/awareness and inter-seizure interval (ISI) is depicted for a non-extreme epileptic state/event (Figure 16C) and an extreme epileptic state/event (Figure 16D), in accordance with one embodiment. As shown in Figure 16C, in a patient with relatively long ISI (*i.e.,* infrequent seizure events) responsiveness/awareness recovers to inter-ictal levels before the next seizure. In contrast, Figure 16D depicts relatively short ISIs (*i.e.,* closely temporally spaced seizures) resulting in an extreme epileptic event/state (*e.g.,* a status epilepticus state) during which responsiveness/awareness remains below inter-ictal levels (*i.e.,* at an impaired responsiveness/awareness value).

Turning now to Figure 17A, a graphical representation of various seizure-related measures/dimensions (y-axis) with respect to time (x-axis) are depicted, in accordance with one or more embodiments. Seizure Severity Index (SSI) 1710 (the product of intensity and duration in this embodiment; extent of spread was not considered) may be determined in reference to the seizure threshold 1725 by calculating the time elapsed between the upward and downward crossings multiplied by the maximum of the curve, above the seizure threshold 1725. Inter-seizure intervals may be calculated as the time elapsed between consecutive up-crossings of the seizure threshold or between a down-crossing of the seizure threshold 1725 and the next up-crossing. In addition to, or alternatively, any SSI, other indices such as responsiveness, awareness, kinetic data (*e.g.,* body falls, limb direction, acceleration and trajectory), time spent in and magnitude of relative or absolute tachycardia (or bradycardia) hypoxia, lactic acidosis, and hypercortisolism respiratory failure, cardiac failure, pulmonary edema, cardiac arrhythmias, liver and/or renal failure, arterial hypertension, tissue hypercarbia and/or the like may be depicted. It should be noted that while only one seizure (the first) crosses the status epilepticus threshold 1720, the figure represents an extreme epileptic state due in addition to the increasing progressive depression of responsiveness/awareness and the short ISIs.

As a general matter, a patient's responsiveness and/or awareness, during and/or after an epileptic event or extreme epileptic event may be used as an indicator of epileptic event severity as well as a patient's health and/or disease state. A patient's responsiveness and/or awareness may be measured automatically using a variety of tests that rely on using audio, visual, olfactory or tactile signals to quantify its changes during the ictal and post-ictal periods compared inter-ictal. The responsiveness/awareness 1705 of a patient while not having seizures may be graphically viewed as a responsiveness baseline 1714; the responsiveness/awareness crossed an impairment threshold 1715 in the downward direction during a seizure. That is, as responsiveness/awareness 1705 decreases, the impairment threshold 1715 may be reached or crossed. The responsiveness/awareness 1705 of the patient may be determined before, during and/or after a seizure event in order to determine the patient's responsiveness/awareness 1705, the severity and/or intensity of a seizure event, a patient's risk of an extreme epileptic event/state and/or the like. That is, the more marked or longer a period of unresponsiveness, the more extreme the epileptic event or state, as discussed below with respect to Figure 17B.

As depicted in Figure 17A, a seizure threshold 1725 and an extreme seizure event/state threshold 1720 may be set for a patient. The seizure threshold 1725 and the extreme event/state threshold 1720 may be based upon a reference to a non-extreme or to an extreme reference value(s) selected from one or more of the following: the body data collection module 275, the physical fitness/integrity index unit 355, the physical fitness/integrity determination unit 376, the neurologic index unit 510, the autonomic index unit 520, the endocrine index unit 540, the metabolic index unit 542, the tissue stress marker index unit 550, the QOL unit 274, the SSI unit 295, the ISI unit 245, the seizure burden unit 273, the patient seizure impact unit 296, the site(s) of seizure determination unit 293, the seizure spread, duration and intensity determination unit 294, the seizure (extreme seizure) ranking unit 247, the seizure determination module 299, seizure intensity ranking unit 1010, seizure spread ranking unit 1015, inter-seizure interval ranking unit 1025, seizure duration determination unit 1030, post-ictal determination unit 1033, seizure impact ranking unit 1017, seizure burden ranking unit 1018, inter-seizure interval determination unit 1020, seizure duration ranking unit, the extreme seizure event/state detection, quantification and risk determination unit 290 and/or the extreme epileptic event/state confirmation unit 287. In one embodiment, an upward crossing of the seizure threshold 1720 by a seizure metric 1710 may indicate that a patient is having an extreme seizure event. In accordance with one embodiment, a downward crossing of a threshold 1730 by the seizure metric 1710 is indicative of it entering the post-ictal energy zone state 1740 associated with decreased responsiveness/awareness 1705 with complex partial or generalized seizures. As shown in Figure 17A, successive seizure events and/or extreme seizure events (indicated by the upward crossings of the status threshold 1720 and/or the seizure threshold 1725) are associated in this embodiment with increasing impairment of responsiveness/awareness 1705.

Turning now to Figure 17B, a graphical representation of conditional (compared to the unconditional) probability of seizure occurrence as a function of time elapsed from the last seizure event, is depicted according to one or more embodiments.

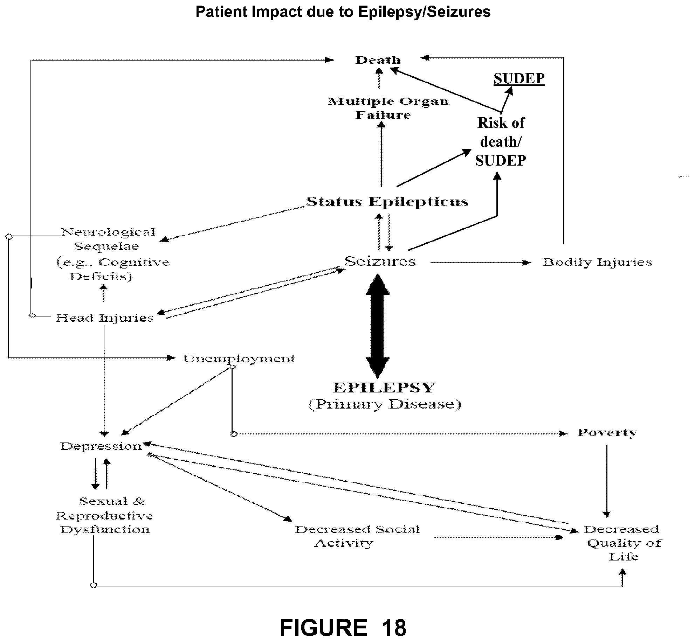

Turning now to Figure 18, a stylized graphical representation of inter-relations of epilepsy as a disease with its symptoms of and effects on patients is provided, in accordance with one or more embodiments.

In one embodiment, identifying an extreme seizure event in a patient may be performed by examining a body index that is affected by a first seizure event. If the change in the body index caused by a seizure does not return to within a range of reference values prior to the occurrence of a second seizure, in one embodiment, this would be indicative of an extreme seizure event. For example, if the heart rate of the patient rises during a first seizure, and the heart rate remains above reference values by the time a second seizure occurs, this seizure is classified as extreme even if the ISI interval duration is within a range of non-extreme reference values for that patient. In this case, the MD 200 may take one or more actions in response to the extreme seizure event, such as issuing a warning (e.g., to a patient, care giver(s) and/or emergency responders), categorizing the event, logging the event, providing therapy(ies), etc. Providing a therapy may include providing an electrical therapy, a chemical therapy, a thermal therapy, a supportive treatment, etc., for treating the seizure event and the changes it causes in body signals. In one embodiment, providing the supportive treatment may include at least one of providing fluids, providing tracheal intubation, providing body cooling, providing brain cooling, providing oxygen, or providing non-seizure drugs. In another embodiment, a seizure may be classified and managed as an extreme event if the seizure occurs in the context of a body signal change not caused by a seizure. For example, a patient has a diagnosis of epilepsy and of cardiac arrhythmia and neither is the cause of the other, that is, they are etiologically unrelated; any seizure that occurs when the patient is having an arrhythmia, would be classified and managed as an extreme event. If a patient with generalized tonic-clonic seizures also has asthma and a seizure occurs during an asthma attack this seizure could be classified and managed as an extreme event.

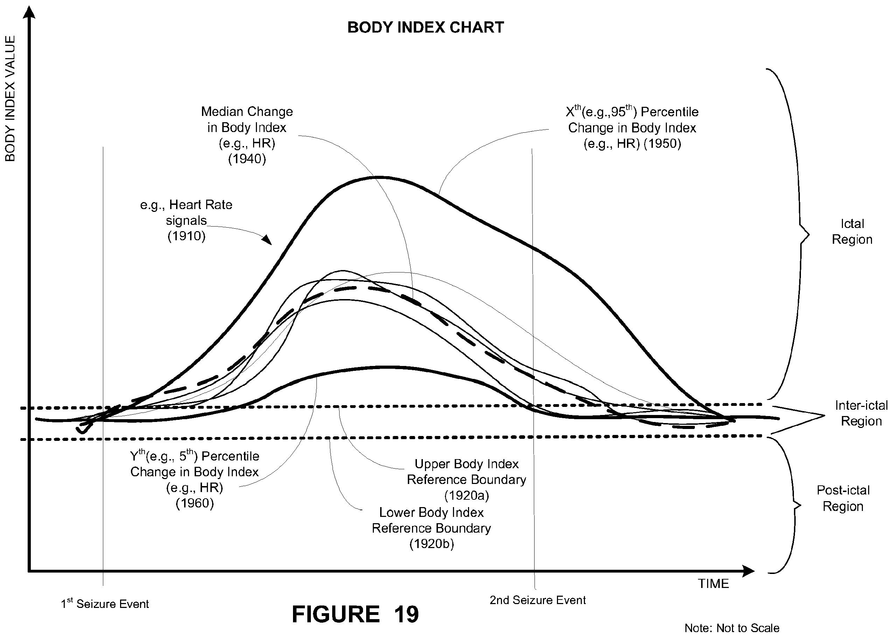

Turning now to Figure 19, a stylized depiction of an exemplary body index chart, in accordance with one embodiment, is illustrated. For ease of discussion, the body index value relating to Figure 19 is described in terms of heart rate; however, the concepts relating to Figure 19 would apply to a variety of body index values.

Prior to the occurrence of a first seizure event, heart rate values for a patient may be within a range of body index reference values indicated by an upper body index reference boundary 1920a, and a lower body index reference boundary 1920b. The range of body index reference values 1920a, 1920b generally relates to an inter-ictal region, as shown in Figure 19. After the occurrence of the first seizure event, heart rate signals 1910 may rise and fall in the ictal region, as exemplified in Figure 19. A dashed-line curve representing the median response of the change in the patient's ictal heart rate (1940), is shown in Figure 19. In time, after the end of the seizure, the heart rate of the patient may generally return to reference values 1920 (inter-ictal region). In one embodiment, deviations from the median change in ictal heart rate 1940 (as determined from a multiplicity of seizure events) may be determined in order to establish whether or not an extreme seizure event has occurred. In one or more embodiments, seizures of similar duration may be compared, a multiplicity of seizures may be normalized and compared, a time warping function may be applied to one or more seizures for comparison of the one or more seizures.

Continuing referring to Figure 19 as an example, changes in heart rate outside an X^{th} percentile (*e.g*., 1950) and a Y^{th} percentile (*e.g.,* 1960) changes in heart rate that occur as a result of the first seizure event may be used to determine whether an extreme seizure event has occurred. In one embodiment, the X^{th} percentile may represent the 95^{th} percentile, or alternatively, various other percentiles (*e.g.,* 90^{th}, 85^{th}, 80^{th}, 75^{th}, 70^{th}, 65^{th}, 60^{th}, 55^{th}, 50^{th}, etc.). Similarly, in one embodiment, the Y^{th} percentile may represent the 5^{th} percentile, or alternatively, various other percentiles (*e.g.,* 10^{th}, 15^{th}, 20^{th}, 25^{th}, 30^{th}, 35^{th}, 40^{th}, 45^{th}, 50^{th}, etc.).

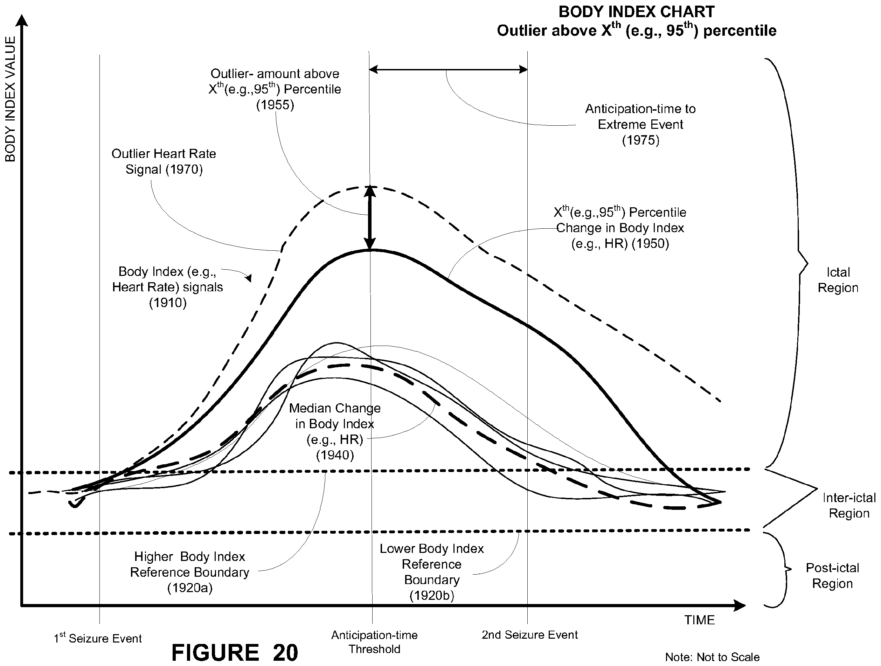

Turning now to Figure 20, a stylized depiction of a body index chart showing an outlier heart rate change, in accordance with one embodiment, is illustrated. As an example, after the occurrence of a first seizure event, a patient's heart rate changes may be above the 95^{th} percentile 1950. An outlier heart rate signal 1970 that rises above the X^{th} percentile change in heart rate 1950 may be determined. Moreover, in one embodiment, if the value of the outlier heart rate signal 1970 exceeds the X^{th} percentile (*e.g*., 95^{th} percentile in this example) change in heart rate 1950 by a predetermined distance (outlier distance from the X^{th} percentile 1955), may indicate an increased probability that the next epileptic event may also be extreme, and that a high-risk time period (to the next epileptic event) is deemed to have been entered. The high-risk time period may refer to an elevated risk of an extreme seizure event occurring before a patient returns to an inter-ictal state. In one embodiment, when the high-risk time period is entered, one or more actions (e.g., issuing a warning, delivering a therapy, logging, disabling a vehicle or power equipment, etc.) may be performed. Further, one or more counter(s) may be used to maintain a count of high-risk time periods, as well as a log of time periods described by the beginning of a high risk time period, until a subsequent seizure. The log may also include time of day of the high risk time period, patient body index data, etc. Such logged information may be used to estimate the time of onset of an extreme event.

In one embodiment, if a second seizure event occurs prior to the detected change in heart rate returning to the inter-ictal range (within the body index reference values 1920), a determination that an extreme seizure condition is occurring or has occurred may be made.

In an alternative embodiment, when the second seizure event is detected, the SSI value of the second seizure event is compared to a reference SSI value. If the SSI value of the second seizure event is greater than a reference SSI value, a determination that an extreme seizure condition is occurring or has occurred may be made.

The recovery time(s) of a body index relative to a subsequent seizure event may provide an indication of an extreme seizure event. In one embodiment, various body index data may be collected for each patient, and reference body index value(s) for a particular patient may be determined. Further, reference body index values for groups or types of patients, seizures (e.g., partial or generalized) or epilepsy (e.g., symptomatic or idiopathic) may be determined. A database for storing various categories of body index data may be provided. This database may be used by the MD 200 to perform the assessment of extreme seizure events described herein. In addition to comparing the body index response to a reference body index, comparison of the body index response may also include comparing the morphology of body index response to a predetermined morphology reference.

In one embodiment, one or more actions may be performed upon a determination that an extreme seizure event has occurred, including but not limited to issuing warning, logging, providing therapy, informing medical personal, disabling one of more devices/machinery (e.g., an electric saw being operated by a patient), etc. Further, the extreme seizure event may be ranked as a result of determining that the change in the detected body signal was above a first threshold (or below a first threshold) when the second seizure event occurred. Similarly, the extreme seizure event may be ranked as a result of determining that the change in the detected body signal was above a second threshold (or below a second threshold) when the second seizure event occurred. In one embodiment, the first ranking is higher than the second ranking.

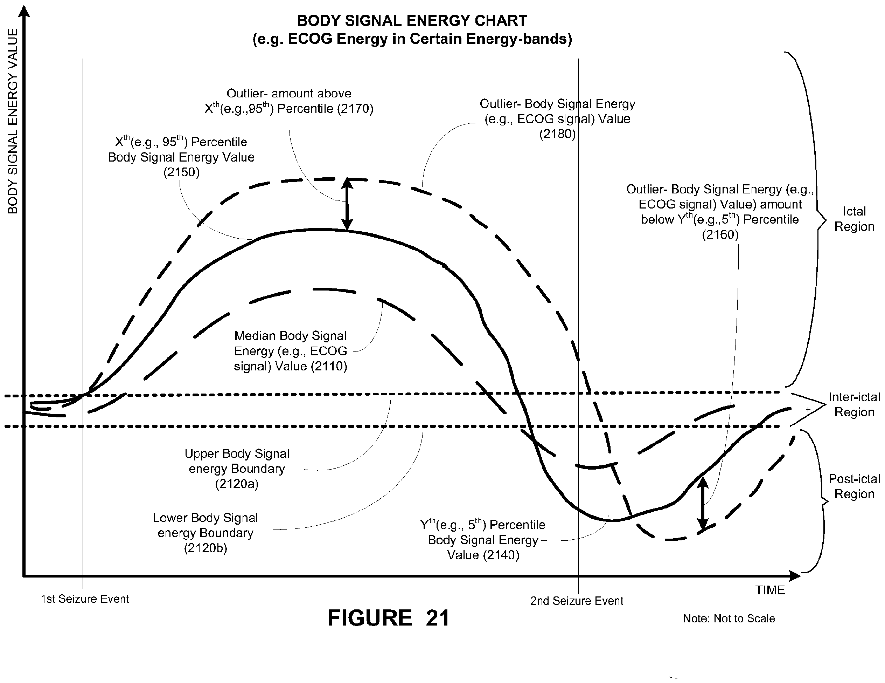

Similar analysis may be performed on other types of body signals, such as, but not limited to, heart activity, respiratory activity, blood pressure, body temperature, blood gas concentrations, and/or ECoG. Turning now to Figure 21, a stylized depiction of a body signal energy chart (*e.g.*, ECoG), in accordance with one embodiment, is illustrated.

Figure 21 illustrates a median body signal energy change (*e.g.*, for an ECOG signal) (2110). The median body signal energy 2110 may increase from a range of reference signal energy values described by the upper and lower body signal energy boundaries 2120a, 2120b (*i.e.*, inter-ictal region) to the ictal region, and when the seizure ends, may enter the post-ictal region (*e.g*., below the reference body signal energy value 2120b). It should be noted in Figure 21 that the body signal energy generally passes transiently through the inter-ictal region and into the post-ictal region. Relative to the median body signal ictal energy change 2110, a response representing an X^{th} percentile change in body signal energy value 2150 and a Y^{th} percentile change in body signal post-ictal energy value 2140 may be determined (as illustrated in Figure 21). In one embodiment, the X^{th} percentile may represent the 95^{th} percentile, or alternatively, various other percentiles (*e.g.*, 90^{th}, 85^{th}, 80^{th}, 75^{th}, 70^{th}, 65^{th}, 60^{th}, 55^{th}, 50^{th}, etc.). Similarly, in one embodiment, the Y^{th} percentile may represent the 5^{th} percentile, or alternatively, various other percentiles (*e.g.*, 10^{th}, 15^{th}, 20^{th}, 25^{th}, 30^{th}, 35^{th}, 40^{th}, 45^{th}, 50^{th}, etc.). Moreover, an outlier body signal energy value 2180 may be tracked relative to the X^{th} percentile change in body signal energy value 2150 and the Y^{th} percentile change in body signal energy value 2140. It should be noted that other measures of central tendency, *e.g*., mean, quartiles, etc., or other statistical measures may be used instead of, or in addition to, the median values exemplarily depicted in Figures 19-22 and as described herein.

In one embodiment, a determination may be made as to whether an outlier body signal energy value (2180) is above an X^{th} percentile by a predetermined margin, i.e., an outlier distance above (2170) an X^{th} percentile. Upon an affirmative determination based upon this inquiry, a determination may be made that an extreme seizure event is occurring or is imminent. Similarly, a determination may be made as to whether an outlier body signal energy value (2170) is below an Y^{th} percentile by a predetermined margin, *i.e.*, an outlier distance below (2140) a Y^{th} percentile. Upon an affirmative determination based upon this inquiry, a determination may be made that an extreme seizure event is occurring or is imminent. In one embodiment, one or more action may be performed upon a determination that an extreme seizure event has occurred or is imminent, including but not limited to issuing warning, logging, providing therapy, informing medical personal, disabling one of more equipment operation, automobile, machinery, etc. In some embodiments, an extreme seizure event may be declared regardless of ISI and/or SSI values if a seizure occurs while the seizure burden and/or patient impact have not returned to inter-ictal values. For example, a subject falls during a seizure and suffers a brain contusion as a result of this. Any seizure that occurs within the period of time before the contusion clears (e.g., six weeks) may be declared as extreme regardless of SSI and/or ISI values based for example, upon the patient's health.

In addition to comparing the detected body signal energy response to reference or median energy signal values, comparison of the body signal energy response may include comparing the morphology of the body signal energy response to a predetermined morphology. In another embodiment, the rate of change (increase or decrease) instead of the magnitude of the change in a body signal may be used to determine of an event is extreme.

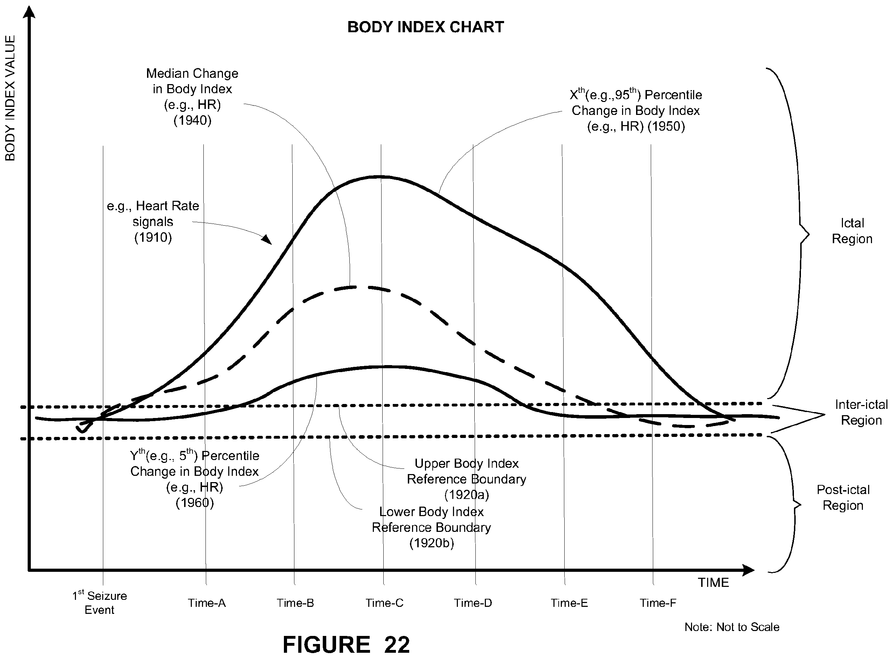

Turning now to Figure 22, an exemplary, stylized depiction of various time periods within distributions (in percentiles) of values of an exemplary body index chart, during the inter-ictal, ictal and postictal periods, is illustrated. As described above, when a second seizure event occurs prior to the time period when the change in body index returns back to the inter-ictal range (1920a, 1920b), an extreme seizure event is deemed to have occurred. In one embodiment, extreme seizure events may be further granulized or ranked. The ranking may, for example be based upon the particular time within the ictal time period of the first seizure at which the second seizure occurs. Figure 22 illustrates various time markers (Time -A through Time -F) that can be used to rank extreme seizure events and the ranking may be performed, for example, by determining the time at which the second seizure occurred relative to the onset of the first seizure, the SSI percentile value of the first seizure at the onset of the second seizure, and for a given percentile value of the first seizure, whether the second seizure occurs near the onset (Time-A), near the peak (Time-C) or near the end (Time-F) of the seizure. For example, if a second seizure occurs at Time-C during a first seizure with an SSI in the 95^{th} percentile , this second seizure will be ranked higher than if the second seizure had occurred at Time-A. Additionally, a second seizure occurring at Time-B may be ranked as more extreme if the SSI value of the first seizure is in the 50^{th} percentile than if it is in the 5^{th} percentile. In this manner, extreme seizure events can be ranked from less to more extreme) based upon time of occurrence of the second seizure (within the ictal period of the first seizure) and/or its SSI value.

In alternative embodiments, in addition to the ISI and SSI determinations described above, other indices may be used to determine whether an extreme seizure event has taken place or is imminent. For example, pathologic high frequency brain wave oscillations may be used as an indicator that an extreme seizure event has taken place or is imminent. In one embodiment, pathologic high frequency oscillations may be described as neuronal/neuropil oscillations with a frequency of approximately 80 Hz or higher.

Other exemplary markers used to determine whether an extreme seizure event has taken place or is imminent may be based upon analysis of epileptiform discharge(s), electrodecremental response(s), and electrographic seizures. An electrographic seizure may refer to a sub-clinical seizure that is generally only detectable using EEG or other electrical measurements. In one embodiment, after a threshold number (*e.g*., two seizures within an ISI below the 20^{th} percentile of a distribution of an ISI value) of electrographic seizures have occurred within a predetermined time period, a determination may be made whether an extreme seizure event has taken place or is imminent. Further, electrodecremental response may refer to sudden transient decreases in the amplitude of background EEG rhythms (*e.g*., recorded using conventional filter settings such as 1Hz and 70Hz) associated with the appearance of behavioral activity indicative of seizures. In one embodiment, transient decreases in the amplitude of background EEG rhythms and/or appearance of activity indicative of seizures (e.g., seizure activity detected by sensors such as an accelerometer, inclinometer, sound detector, etc.) may be used to determine whether an extreme seizure event has taken place or is imminent. Moreover, epileptiform discharge may refer to brain shape, e.g., sharply contoured brain wave(s) with a duration of about up to 200 ms, that may or may not form complexes with slow waves (e.g., 1-4 Hz). In one embodiment, the duration of the epileptiform discharge, site of origin, and/or the shape, e.g., sharpness of the brain waves may be used to determine whether an extreme seizure event is imminent, is taking place, or has taken place.

## Claims

1. A computer implemented method for identifying an extreme seizure event in a patient, comprising:
determining at least one of an autonomic index, a neurologic index, a metabolic index, an endocrine index, or a tissue stress index, said at least one determined index being based upon body data;
identifying a seizure event based upon said at least one determined index;
determining at least one seizure severity index, SSI, value indicative of the severity of said seizure event based on the product of the duration of the seizure event determined as the time that any of the values of the autonomic index, neurologic index, metabolic index, endocrine index, or tissue stress index differ from inter-ictal and/or post-ictal values and the peak intensity of the seizure event defined by a maximum value of the body data;
comparing said determined at least one SSI value to at least one extreme reference value, wherein the extreme reference value is a value above the ninetieth percentile of a plurality of past SSI values over a first time period; and
identifying an occurrence of an extreme seizure event, based upon the comparison of said determined SSI value to said at least one extreme reference value;
and wherein said extreme seizure event comprises at least one of a status epilepticus event or a pathophysiological effect resulting from an extreme epileptic state, the pathophysiological effect being selected from the group consisting of:
damage to brain tissue resulting in permanent/serious motor/visual/sensory/cognitive skills; respiratory failure, cardiac failure, pulmonary edema, cardiac arrhythmia, arterial blood acidosis, liver/renal failure, bed sores, bone fractures, abrasions, bruises, organ failure, multi- organ failure, arterial hypertension, tissue hypoxia and tissue acidosis.

2. The method of claim 1, further comprising performing at least one action in response to identifying the occurrence of an extreme seizure event, said action comprising at least one of:
issuing a warning in response to said identifying based upon said comparing of said determined at least one SSI value to said at least one extreme reference value.

3. The method of claim 2, further comprising initiating a logging sequence of said extreme seizure event, wherein said logging sequence further comprises
storing into a memory of a medical device or a database coupled to said medical device at least one of:
said SSI value, a start time of said extreme seizure event, an end time of said extreme seizure event, a duration of said extreme seizure event, a time of issuing said warning into a memory of at least one of a medical device and a database operatively coupled to said medical device, a ranking of the SSI value, a characterization of the SSI value compared to at least one SSI value for a prior seizure event, and an activity signal indicative of the patient's activity state during a time period proximate to said seizure event.

4. The method of claim 2, further comprising:
initiating an extreme seizure event confirmation of said identifying an occurrence of an extreme seizure event;
receiving a response to said initiating; and
performing providing a signal adapted to confirm or negate the extreme seizure event determination.

5. The method of claim 1 further comprising
a) performing at least one of ranking the identified extreme seizure event in reference to at least one prior extreme event or determining the time elapsed since at least one of a plurality of prior extreme seizure events; and
b) identifying a time spent in a state of an extreme seizure event over a time window, wherein the time window is at least one of a microscopic, a mesoscopic or a macroscopic time window.

6. The method of claim 1, wherein the extreme reference value comprises a value beyond 2.5 standard deviations to the right or left of the mean for a normal or a normalized distribution.

7. The method of claim 1, wherein said at least one SSI value is based upon at least one data set of seizure metric data related to a seizure event, said seizure metric data relating to a time period, wherein said at least one data set of seizure metric data is based at least upon said body data.

8. An apparatus, comprising:
a determination component adapted to:
determine at least one of an autonomic index, a neurologic index, a metabolic index, an endocrine index, or a tissue stress index, said at least one determined index being based upon body data;
identify a seizure event based upon said at least one determined index;
determine an SSI value indicative of the severity of said seizure event based on the product of the duration of the seizure event determined as the time that any of the values of the autonomic index, neurologic index, metabolic index, endocrine index, or tissue stress index differ from inter-ictal and/or post-ictal values and the peak intensity of the seizure event defined by a maximum value of the body data;
compare the determined SSI value to at least one extreme reference value, wherein the extreme reference value is a value above the ninetieth percentile of a plurality of past SSI values over a first time period, and
identify an occurrence of an extreme epileptic event, based upon the comparison of said determined SSI value to said at least one extreme reference value;
and wherein said extreme seizure event comprises at least one of a status epilepticus event or a pathophysiological effect resulting from an extreme epileptic state, the pathophysiological effect being selected from the group consisting of:
damage to brain tissue resulting in permanent/serious motor/visual/sensory/cognitive skills; respiratory failure, cardiac failure, pulmonary edema, cardiac arrhythmia, arterial blood acidosis, liver/renal failure, bed sores, bone fractures, abrasions, bruises, organ failure, multi- organ failure, arterial hypertension, tissue hypoxia and tissue acidosis.

9. The apparatus of claim 8, wherein said determination component further comprises a controller, said controller being adapted to:
control one or more operations of said apparatus;
process at least one of internal data or external data, said at least one of internal data or external data being associated with identifying an occurrence of an extreme epileptic event;
store data, said data comprising at least one of internal data, external data or processed data;
set a flag indicative of said extreme epileptic event;
provide a signal indicative of said extreme epileptic event;
provide a therapy based upon said extreme epileptic event;
issue a warning based upon said signal indicative of said extreme epileptic event;
determine a ranking of said extreme epileptic event compared to one or more previous extreme epileptic events;
initiate at least one of a logging sequence and a reporting sequence related to said extreme epileptic event; and
transmit a stored portion of data related to said extreme epileptic event to at least one of an external device or an external entity.

10. The apparatus of claim 8 or 9, further comprising a memory adapted to:
store said data, said data comprising at least one of internal data, external data or processed data;
store said warning;
store said ranking; and
store a value indicative of the time spent in an extreme epileptic event.

11. The apparatus of claim 8, 9 or 10, further comprising a seizure determination module adapted to detect a seizure and determine at least one characteristic of the detected seizure.

12. The apparatus of one of the claims 8 to 11, further comprising a seizure severity index, SSI, unit adapted to determine a value of an SSI.

13. The apparatus of one of the claims 8 to 12, further comprising an extreme epileptic event unit adapted to perform at least one of determining the presence of an extreme epileptic event or quantifying a risk of an extreme epileptic event.

14. The apparatus of claim 9, wherein said at least one SSI value is based upon at least one data set of seizure metric data related to a seizure event, said seizure dataset related to a time period, wherein said at least one data set of seizure metric data is based at least upon said body data.

## Patentansprüche

1. Ein computerimplementiertes Verfahren zum Identifizieren eines extremen Anfallsereignisses eines Patienten, umfassend:
Bestimmen zumindest eines von einem autonomen Index, einem neurologischen Index, einem metabolischen Index, einem endokrinen Index oder einem Gewebestressindex, wobei der genannte zumindest eine bestimmte Index auf Körperdaten basiert;
Identifizieren eines Anfallsereignisses auf der Grundlage des genannten zumindest einen bestimmten Indexes;
Bestimmen zumindest eines Anfallsstärkeindex, SSI, der die Stärke des genannten Anfallsereignisses auf der Grundlage des Produkts von der Zeitdauer des Anfallsereignisses, die als die Zeitdauer bestimmt wird, in der sich einer der Werte des autonomen Indexes, neurologischen Indexes, metabolischen Indexes, endokrinen Indexes oder Gewebestressindexes von den Zwischenanfalls- und/oder Nachanfallswerten unterscheidet, und der maximalen Intensität des Anfallsereignisses, wie sie durch einen Maximalwert der Körperdaten definiert wird, anzeigt;
Vergleichen des genannten zumindest einen SSI-Werts mit zumindest einem extremem Referenzwert, wobei der extreme Referenzwert ein Wert oberhalb des neunten Perzintels einer Mehrzahl an früheren SSI-Werten über eine erste Zeitdauer ist; und
Identifizieren eines Auftretens eines extremen Anfallsereignisses auf der Grundlage des Vergleichs des genannten bestimmten SSI-Werts mit dem genannten zumindest einen extremen Referenzwert;
und wobei das extreme Anfallsereignis zumindest eines von einem Status Epilepticus oder einem pathophysiologischen Effekt, der aus einem extremen epileptischen Zustand resultiert, umfasst, wobei der pathophysiologischen Effekt aus der Gruppe ausgewählt wird, die besteht aus:
Beschädigung von Gehirngewebe, die in permanenten/schweren motorischen/visuellen/sensorischen/kognitiven Fertigkeiten resultiert, respiratorischen Ausfällen, kardialen Ausfällen, Lungenödem, Herzrhythmusstörungen, arterieller Blutübersäuerung, Leber-/Nierenausfall, Dekubitus, Knochenbrüchen, Hautabschürfungen, Prellungen, Organversagen, multiples Organversagen, arteriellen Bluthochdruck, Gewebehypoxie und Gewebeübersäuerung.

2. Das Verfahren von Anspruch 1, weiterhin das Ausführen zumindest einer Aktion in Reaktion auf das Identifizieren des Auftretens eines extremen Anfallsereignisses umfassend, wobei die Aktion zumindest eines von dem Folgenden umfasst:
Ausgeben einer Warnung in Reaktion auf die genannte Identifikation basierend auf dem genannten Vergleichen des genannten bestimmten zumindest einen SSI-Wertes mit dem genannten zumindest einen extremen Referenzwert.

3. Das Verfahren von Anspruch 2, weiterhin ein Initiieren einer Protokollsequenz des genannten extremen Anfallsereignisses umfassend, wobei die genannte Protokollsequenz weiterhin umfasst
Speichern in den Speicher einer medizinischen Vorrichtung oder einer Datenbank, die mit der medizinischen Vorrichtung verbunden ist, zumindest eines von:
dem genannten SSI-Wert, einer Startzeit des extremen Anfallsereignisses, einer Endzeit des extremen Anfallsereignisses, einer Dauer des extremen Anfallsereignisses, einer Zeit, zu der die genannte Warnung eingegeben wird, in einen Speicher zumindest eines von einer medizinischen Vorrichtung und einer Datenbank, die mit der medizinischen Vorrichtung operativ verbunden ist, einer Rangordnung des SSI-Werts, einer Charakterisierung des SSI-Werts verglichen mit zumindest einem SSI-Wert eines vorhergehenden Anfallsereignisses und einem Aktivitätssignal, das den Aktivitätszustand des Patienten während einer Zeitdauer nahe dem genannten Anfallsereignis anzeigt.

4. Das Verfahren von Anspruch 2, das weiterhin umfasst:
Initiieren einer Bestätigung eines extremen Anfallsereignisses für das genannte Identifizieren eines Auftretens eines extremen Anfallsereignisses;
Empfangen einer Antwort auf das genannte Initiieren; und
Ausführen des Lieferns eines Signals, das geeignet ist, die Bestimmung des extremen Anfallsereignisses zu bestätigen oder negieren.

5. Das Verfahren von Anspruch 1, das weiterhin umfasst
a) Ausführen von zumindest einem von einem Erstellen einer Rangordnung des identifizierten extremen Anfallsereignisses mit Bezug auf zumindest ein vorheriges extremes Anfallsereignis oder Bestimmen der Zeit, die seit zumindest einem von mehreren vorherigen extremen Anfallsereignissen vergangen ist; und
b) Identifizieren einer Zeit, die über ein Zeitfenster in einem Zustand eines extremen Anfallsereignisses verbracht wurde, wobei das Zeitfenster zumindest eines von einem mikroskopischen, mesoskopischen oder makroskopischen Zeitfenster ist.

6. Das Verfahren von Anspruch 1, in dem der extreme Referenzwert einen Wert oberhalb von einer Standardabweichung von 2,5 rechts oder links des Mittelwerts einer Normalverteilung oder einer normalisierten Verteilung umfasst.

7. Das Verfahren von Anspruch 1, in dem der genannte zumindest eine SSI-Wert auf zumindest einem Datensatz metrischer Anfallsdaten mit Bezug auf ein Anfallsereignis basiert, wobei sich die metrischen Anfallsdaten auf eine Zeitdauer beziehen, in der der zumindest eine Datensatz metrischer Anfallsdaten zumindest auf den genannten Körperdaten basiert.

8. Eine Vorrichtung, die umfasst:
eine Bestimmungskomponente, die dazu ausgebildet ist:
zumindest eines von einem autonomen Index, einem neurologischen Index, einem metabolischen Index, einem endokrinen Index, oder einem Gewebestressindex zu bestimmen, wobei der genannte zumindest eine bestimmte Index auf Körperdaten basiert;
ein Anfallsereignis auf der Grundlage des genannten zumindest einen bestimmten Indexes zu bestimmen;
einen SSI-Wert zu bestimmen, der die Stärke des genannten Anfallsereignisses auf der Grundlage des Produkts von der Zeitdauer des Anfallsereignisses, die als die Zeit bestimmt wird, in der sich einer der Werte des autonomen Indexes, neurologischen Indexes, metabolischen Indexes, endokrinen Indexes oder Gewebespannungsindexes von den Zwischenanfalls- und/oder Nachanfallswerten unterscheidet, und der maximalen Intensität des Anfallsereignisses, wie sie durch einen Maximalwert der Körperdaten definiert wird, anzeigt;
den genannten zumindest einen SSI-Wert mit zumindest einem extremem Referenzwert zu vergleichen, wobei der extreme Referenzwert ein Wert oberhalb des neunten Perzintels einer Mehrzahl an früheren SSI-Werten über eine erste Zeitdauer ist; und
ein Auftreten eines extremen epileptischen Ereignisses auf der Grundlage des Vergleichs des genannten bestimmten SSI-Werts mit dem genannten zumindest einen extremen Referenzwert zu identifizieren;
und wobei das extreme Anfallsereignis zumindest eines von einem Status Epilepticus oder einem pathophysiologischen Effekt, der aus einem extremen epileptischen Zustand resultiert, umfasst, wobei der pathophysiologischen Effekt aus der Gruppe ausgewählt wird, die besteht aus:
Beschädigung von Gehirngewebe, die in permanenten/schweren motorischen/visuellen/sensorischen/kognitiven Fertigkeiten resultiert, respiratorischen Ausfällen, kardialen Ausfällen, Lungenödem, Herzrhythmusstörungen, arterieller Blutübersäuerung, Leber-/Nierenausfall, Dekubitus, Knochenbrüchen, Hautabschürfungen, Prellungen, Organversagen, multiples Organversagen, arteriellen Bluthochdruck, Gewebehypoxie und Gewebeübersäuerung.

9. Die Vorrichtung von Anspruch 8, in der die genannte Bestimmungskomponente einen Kontroller umfasst, wobei der genannte Kontroller dazu ausgebildet ist:
eine oder mehrere Operationen der Vorrichtung zu steuern;
zumindest eines von internen Daten und externen Daten zu verarbeiten, die mit einem Identifizieren eines Auftretens eines extremen epileptischen Ereignisses assoziiert sind;
Daten zu speichern, wobei die genannten Daten zumindest eines von internen Daten, externen Daten oder verarbeiteten Daten umfassen;
eine Flag zu setzen, die das genannte extreme epileptische Ereignis anzeigt;
ein Signal zu liefern, das das genannte extreme epileptische Ereignis anzeigt;
basierend auf dem extremen epileptischen Ereignis eine Therapie zu verabreichen;
basierend auf dem genannten Signal, das das genannte extreme epileptische Ereignis anzeigt, eine Warnung auszugeben;
eine Rangordnung des genannten extremen epileptischen Ereignisses im Vergleich zu einem oder mehreren vorherigen extremen epileptischen Ereignissen zu bestimmen;
zumindest eines von einer Protokollsequenz und einer Berichtssequenz bezogen auf das genannte extreme epileptische Ereignis zu initiieren; und
einen gespeicherten Teil von Daten, der sich auf das genannte extreme epileptische Ereignis bezieht, zumindest zu einem von einer externen Vorrichtung und einer externen Einheit zu senden.

10. Die Vorrichtung von Anspruch 8 oder 9, die weiterhin einen Speicher umfasst, der dazu ausgebildet ist:
die genannten Daten zu speichern, wobei die genannten Daten zumindest eines von internen Daten, externen Daten und verarbeiteten Daten umfassen;
die genannte Warnung zu speichern;
die genannte Rangordnung zu speichern; und
einen Wert zu speichern, der die Zeitdauer des extremen epileptischen Ereignisses angibt.

11. Die Vorrichtung von Anspruch 8, 9 oder 10, weiterhin ein Anfallsbestimmungsmodul umfassend, das dazu ausgebildet ist, einen Anfall zu detektieren und zumindest eine Charakteristik des detektierten Anfalls zu bestimmen.

12. Die Vorrichtung von einem der Ansprüche 8 bis 11, weiterhin eine Anfallsstärkeindex, SSI, - Einheit umfassend, die dazu ausgebildet ist, einen Wert eines SSI zu bestimmen.

13. Die Vorrichtung von einem der Ansprüche 8 bis 12, weiterhin eine Extremes-Epileptisches-Ereignis-Einheit umfassend, die dazu ausgebildet ist, zumindest eines von einem Bestimmen des Vorliegens eines extremen epileptischen Ereignisses oder Quantifizieren eines Risikos für ein extremes epileptisches Ereignis auszuführen.

14. Die Vorrichtung von Anspruch 9, wobei der genannte zumindest eine SSI-Wert auf zumindest einem Datensatz metrischer Anfallsdaten mit Bezug auf ein Anfallsereignis basiert, wobei sich der Anfallsdatensatz auf eine Zeitdauer bezieht, in der der zumindest eine Datensatz metrischer Anfallsdaten zumindest auf den genannten Körperdaten basiert.

## Revendications

1. Procédé mis en œuvre sur ordinateur permettant d'identifier un événement de crise d'épilepsie extrême chez un patient, comprenant :
la détermination d'au moins l'un parmi un indice de fonctionnement autonome, un indice neurologique, un indice métabolique, un indice endocrinien, un indice de contrainte des tissus, ledit ou lesdits indices déterminés étant fondés sur des données corporelles,
l'identification d'un événement de crise d'épilepsie fondé sur ledit ou lesdits indices déterminés,
la détermination d'au moins un indice de sévérité de crise d'épilepsie, SSI, indicatif de la sévérité dudit événement de crise, fondé sur le produit de la durée de l'événement de crise déterminée comme le temps pendant lequel l'une quelconque des valeurs de l'indice de fonctionnement autonome, l'indice neurologique, l'indice métabolique, l'indice endocrinien ou l'indice de contrainte des tissus diffère de valeurs entre crises et/ou après crise ; ainsi que de l'intensité crête de l'événement de crise d'épilepsie défini par une valeur maximale des données corporelles,
la comparaison de ladite ou desdites valeurs d'indices SSI à au moins une valeur de référence extrême, la valeur de référence extrême étant une valeur située au-dessus du quatre-vingt-dixième percentile d'une pluralité de valeurs passées d'indices SSI sur un premier intervalle de temps, et
l'identification de l'occurrence d'un événement de crise d'épilepsie extrême sur la base de la comparaison de ladite valeur déterminée d'indice SSI à ladite ou aux dites valeurs de référence extrêmes,
et dans lequel ledit événement de crise d'épilepsie extrême comprend au moins l'un d'un événement d'état de mal épileptique ou d'un effet patho-physiologique résultant d'un état épileptique extrême, l'effet patho-physiologique étant sélectionné à partir du groupe constitué de :
lésion sur le tissu cérébral résultant en des aptitudes motrices / visuelles / sensitives / cognitives permanentes ou sérieuses ; défaillance respiratoire, défaillance cardiaque, œdème pulmonaire, arythmie cardiaque, acidose du sang artériel, défaillance hépatique ou rénale, escarres, fractures osseuses, abrasions, contusions, défaillance d'un organe, défaillance de plusieurs organes, hypertension artérielle, hypoxie des tissus et acidose des tissus.

2. Procédé selon la revendication 1, comprenant en outre l'exécution d'au moins une action en réponse à l'identification de l'occurrence d'un événement de crise d'épilepsie extrême, ladite action comprenant au moins l'une parmi :
l'émission d'une alarme en réponse à ladite identification sur la base de ladite comparaison de ladite ou desdites valeurs d'indices SSI à ladite ou aux dites valeurs de référence extrêmes.

3. Procédé selon la revendication 2, comprenant en outre l'initiation d'une séquence de consignation dudit événement de crise d'épilepsie extrême, ladite séquence de consignation comprenant en outre :
le stockage, dans une mémoire de dispositif médical ou dans une base de données couplée au dit dispositif médical, d'au moins un élément parmi :
ladite valeur d'indice SSI, l'instant de début dudit événement de crise d'épilepsie extrême, l'instant de fin dudit événement d'épilepsie extrême, la durée dudit événement d'épilepsie extrême, l'instant d'émission de ladite alarme dans une mémoire d'au moins l'un d'un dispositif médical et d'une base de données couplée fonctionnellement au dit dispositif médical, l'ordonnancement de la valeur d'indice SSI, la caractérisation de la valeur d'indice SSI par comparaison à au moins une valeur d'indice SSI pour un événement précédent de crise d'épilepsie, ainsi qu'un signal d'activité indicatif de l'état d'activité du patient pendant un intervalle de temps proche dudit événement de crise d'épilepsie.

4. Procédé selon la revendication 2, comprenant en outre :
le lancement d'une confirmation d'événement de crise d'épilepsie extrême concernant ladite identification de l'occurrence d'un événement de crise d'épilepsie extrême,
la réception d'une réponse au dit lancement, et
l'exécution de la délivrance d'un signal adapté pour confirmer ou bien nier la détermination de l'événement de crise d'épilepsie extrême.

5. Procédé selon la revendication 1, comprenant en outre :
a) l'exécution d'au moins l'un parmi l'ordonnancement de l'événement identifié de crise d'épilepsie extrême par référence à au moins un événement extrême précédent, ou bien la détermination du temps écoulé depuis au moins l'un d'une pluralité d'événements de crise d'épilepsie extrême, et
b) l'identification du temps passé dans un état de crise d'épilepsie extrême sur une fenêtre de temps, la fenêtre de temps étant au moins l'une d'une fenêtre de temps microscopique, mésoscopique ou macroscopique.

6. Procédé selon la revendication 1, dans lequel la valeur de référence extrême comprend une valeur située au-delà de 2,5 écarts type vers la droite ou la gauche de la moyenne pour une distribution normale ou normalisée.

7. Procédé selon la revendication 1, dans lequel ladite ou lesdites valeurs d'indices SSI sont fondées sur au moins un ensemble de données constitué de données de mesure de crise d'épilepsie se rapportant à un événement de crise d'épilepsie, lesdites données de mesure de crise d'épilepsie se rapportant à un intervalle de temps, ledit ou lesdits ensembles de données de mesure de crise d'épilepsie étant fondés au moins sur lesdites données corporelles.

8. Appareil comprenant :
un composant de détermination conçu pour :
déterminer au moins l'un d'un indice de fonctionnement autonome, d'un indice neurologique, d'un indice métabolique, d'un indice endocrinien ou d'un indice de contrainte des tissus, ledit ou lesdits indices déterminés étant fondés sur des données corporelles,
identifier un événement de crise d'épilepsie sur la base dudit ou desdits indices déterminés,
déterminer une valeur d'indice SSI indicative de la sévérité dudit événement de crise d'épilepsie sur la base du produit de la durée de l'événement de crise d'épilepsie déterminé comme étant le temps pendant lequel l'une quelconque des valeurs de l'indice de fonctionnement autonome, l'indice neurologique, l'indice métabolique, l'indice endocrinien ou l'indice de contrainte des tissus diffère de valeurs entre crises et/ou après crise, ainsi que l'intensité crête de l'événement de crise d'épilepsie défini par une valeur maximale des données corporelles,
comparer la valeur déterminée d'indice SSI à au moins une valeur de référence extrême, la valeur de référence extrême étant une valeur située au-dessus du quatre-vingt-dixième percentile d'une pluralité de valeurs passées d'indices SSI sur un premier intervalle de temps, et
identifier l'occurrence d'un événement épileptique extrême sur la base de la comparaison de ladite valeur déterminée d'indice SSI à ladite ou aux dites valeurs de référence extrêmes,
et où ledit événement de crise d'épilepsie extrême comprend au moins l'un d'un événement d'état de mal épileptique ou d'un effet patho-physiologique résultant d'un état épileptique extrême, l'effet patho-physiologique étant sélectionné à partir du groupe constitué de :
lésion sur le tissu cérébral résultant en des aptitudes motrices / visuelles / sensitives / cognitives permanentes ou sérieuses ; défaillance respiratoire, défaillance cardiaque, œdème pulmonaire, arythmie cardiaque, acidose du sang artériel, défaillance hépatique ou rénale, escarres, fractures osseuses, abrasions, contusions, défaillance d'un organe, défaillance de plusieurs organes, hypertension artérielle, hypoxie des tissus et acidose des tissus.

9. Appareil selon la revendication 8, dans lequel ledit composant de détermination comprend en outre un contrôleur, ledit contrôleur étant conçu pour :
piloter un ou plusieurs opérations dudit appareil,
traiter au moins un élément parmi des données internes ou des données externes, ledit ou lesdits éléments parmi les données internes ou les données externes étant associés à l'identification de l'occurrence d'un événement épileptique extrême,
stocker les données, lesdites données comprenant au moins un élément parmi les données internes, les données externes ou des données traitées,
positionner un drapeau indicatif dudit événement épileptique extrême,
délivrer un signal indicatif dudit événement épileptique extrême,
procurer une thérapie sur la base dudit événement épileptique extrême,
émettre une alarme sur la base dudit signal indicatif dudit événement épileptique extrême,
déterminer un ordonnancement dudit événement épileptique extrême par comparaison à un ou plusieurs événements épileptiques extrêmes précédents,
lancer au moins l'une d'une séquence de consignation et d'une séquence de notification se rapportant au dit événement épileptique extrême, et
transmettre une partie stockée des données se rapportant au dit événement épileptique extrême à au moins l'un d'un dispositif externe ou d'une entité externe.

10. Appareil selon la revendication 8 ou la revendication 9, comprenant en outre une mémoire conçue pour :
mémoriser lesdites données, lesdites données comprenant au moins un élément parmi les données internes, les données externes ou les données traitées,
mémoriser ladite alarme,
mémoriser ledit ordonnancement, et
mémoriser une valeur indicative du temps passé pendant un événement épileptique externe.

11. Appareil selon la revendication 8, la revendication 9 ou la revendication 10, comprenant en outre un module de détermination de crise d'épilepsie conçu pour détecter une crise d'épilepsie et pour déterminer au moins une caractéristique de la crise d'épilepsie détectée.

12. Appareil selon l'une des revendications 8 à 11, comprenant en outre une unité d'indexage de sévérité de crise d'épilepsie, SSI, conçue pour déterminer une valeur d'indice SSI.

13. Appareil selon l'une des revendications 8 à 12, comprenant en outre une unité d'événement épileptique extrême conçue pour effectuer au moins l'une de la détermination de la présence d'un événement épileptique extrême ou de la quantification du risque d'un événement épileptique extrême.

14. Appareil selon la revendication 9, dans lequel ladite ou lesdites valeurs d'indices SSI sont fondées sur au moins un ensemble de données constitué de données de mesure de crise d'épilepsie se rapportant à un événement de crise d'épilepsie, ledit ensemble de données de crise d'épilepsie se rapportant à un intervalle de temps, ledit ou lesdits ensembles de données de mesure de crise d'épilepsie étant fondés au moins sur lesdites données corporelles.
